# EUROPÄISCHE PATENTSCHRIFT

(11) **EP 2 301 091 B1**
(45) Veröffentlichungstag und Bekanntmachung des Hinweises auf die Patenterteilung: **21.08.2013**
(21) Anmeldenummer: 09765856.1
(22) Anmeldetag: 17.06.2009
(51) Int. Cl.: H01L 51/30, C07F 9/6581

(54) **VERWENDUNG VON CYCLISCHEN PHOSPHAZENVERBINDUNGEN IN ORGANISCHEN LEUCHTDIODEN**
USE OF CYCLIC PHOSPHAZENE COMPOUNDS IN ORGANIC LIGHT EMITTING DIODES
UTILISATION DE COMPOSES PHOSPHAZÈNE CYCLIQUES DANS DES DIODES ÉLECTROLUMINESCENTES ORGANIQUES

(30) Priorität: 20.06.2008 EP 08158669
(43) Veröffentlichungstag der Anmeldung: 30.03.2011
(73) Patentinhaber: BASF SE, 67056 Ludwigshafen (DE); Osram Opto Semiconductors Gmbh, 93055 Regensburg (DE); Philips Intellectual Property & Standards GmbH, 20099 Hamburg (DE); Koninklijke Philips Electronics N.V., 5621 BA Eindhoven (NL)
(72) Erfinder: FUCHS, Evelyn, 68199 Mannheim (DE); MOLT, Oliver, 69469 Weinheim (DE); LANGER, Nicolle, 64646 Heppenheim (DE); LENNARTZ, Christian, 67105 Schifferstadt (DE); STROHRIEGL, Peter, 95448 Bayreuth (DE); SCHROEGEL, Pamela, 95447 Bayreuth (DE); BOERNER, Herbert Friedrich, 52078 Aachen (DE); HUNZE, Arvid, 91058 Erlangen (DE); KRAUSE, Ralf, 91058 Erlangen (DE); VAN ELSBERGEN, Volker, 52080 Aachen (DE); SCHMID, Günter, 91334 Hemhofen (DE)
(74) Vertreter: Hollah, Dorothee
(86) Internationale Anmeldenummer: PCT/EP2009/057505
(87) Internationale Veröffentlichungsnummer: WO 2009/153276

(56) Entgegenhaltungen:
- CH-A- 493 563
- DE-B- 1 171 916
- JP-A- 2002 265 614
- BOLINK ET AL: "Efficient blue emitting organic light emitting diodes based on fluorescent solution processable cyclic phosphazenes" ORGANIC ELECTRONICS, ELSEVIER, AMSTERDAM, NL, Bd. 9, Nr. 2, 25. Oktober 2007 (2007-10-25), Seiten 155-163, XP022489968 ISSN: 1566-1199
- H. J. BOLINK, S. G. SANTAMARIA, S. SUNDARRAJ, C. ZHEN, A. SELLINGER: "Solution processable phosphorescent dendrimers based on cyclic phosphazenes for use in organic light emitting diodes (OLEDs)" CHEM. COMMUN., 2008, - 4. Dezember 2007 (2007-12-04) Seiten 618-620, XP002542457

## Beschreibung

Die vorliegende Erfindung betrifft eine organische Leuchtdiode, enthaltend mindestens eine cyclische Phosphazenverbindung, eine Licht-emittierende Schicht aufgebaut aus mindestens einem Matrixmaterial und mindestens einem Emittermaterial, wobei das mindestens eine Matrixmaterial mindestens eine cyclische Phosphazenverbindung enthält, die Verwendung von cyclischen Phosphazenverbindungen in organischen Leuchtdioden sowie eine Vorrichtung ausgewählt aus der Gruppe bestehend aus stationären Bildschirmen, mobilen Bildschirmen und Beleuchtungseinheiten enthaltend mindestens eine erfindungsgemäße organische Leuchtdiode

In organischen Leuchtdioden (OLED) wird die Eigenschaft von Materialien ausgenutzt, Licht zu emittieren, wenn sie durch elektrischen Strom angeregt werden. OLEDs sind insbesondere interessant als Alternative zu Kathodenstrahlröhren und Flüssigkristall-displays zur Herstellung von Flachbildschirmen. Aufgrund der sehr kompakten Bauweise und des intrinsisch niedrigen Stromverbrauchs eignen sich die Vorrichtungen enthaltend OLEDs insbesondere für mobile Anwendungen, z. B. für Anwendungen in Handys, Laptops, usw. sowie zur Beleuchtung.

Die Grundprinzipien der Funktionsweise von OLEDs sowie geeignete Aufbauten (Schichten) von OLEDs sind z. B. in WO 2005/113704 und deren zitierten Literatur genannt.

Als Licht-emittierende Materialien (Emitter) können neben fluoreszierenden Materialien (Fluoreszenz-Emitter) phosphoreszierende Materialien (Phosphoreszenz-Emitter) eingesetzt werden. Bei den Phosphoreszenz-Emittern handelt es sich üblicherweise um metallorganische Komplexe, die im Gegensatz zu den Fluoreszenz-Emittern, die eine Singulett-Emission zeigen, eine Triplett-Emission zeigen (M. A. Baldo et al., Appl. Phys. Lett. 1999, 75, 4 - 6). Aus quantenmechanischen Gründen ist bei Verwendung der Phosphoreszenz-Emitter eine bis zu vierfache Quanten-, Energie- und Leistungseffizienz möglich.

Von besonderem Interesse sind organische Leuchtdioden mit langer operativer Lebensdauer, guter Effizienz, hoher Stabilität gegenüber Temperaturbelastung und einer niedrigen Einsatz- und Betriebsspannung, die insbesondere Licht im blauen Bereich des elektromagnetischen Spektrums emittieren.

Um die vorstehend erwähnten Eigenschaften in die Praxis umzusetzen, ist es nicht nur erforderlich, geeignete Emittermaterialien bereitzustellen, sondern auch die anderen Komponenten der OLED (Komplementärmaterialien) müssen in geeigneten Device-Kompositionen aufeinander abgestimmt sein. Solche Device-Kompositionen können zum Beispiel spezielle Matrixmaterialien enthalten, in denen der eigentliche Lichtemitter in verteilter Form vorliegt. Des Weiteren können die Kompositionen Blockermaterialien enthalten, wobei Loch-, Excitionen- und/oder Elektronenblocker in den Device-Kompositionen vorliegen können. Daneben oder alternativ können die Device-Kompositionen des Weiteren Loch-Injektionsmaterialien und/oder ElektronenInjektionsmaterialien und/oder Ladungstransportmaterialien wie Lochleitermaterialien und/oder Elektronenleitermaterialien aufweisen. Dabei hat die Auswahl der vorstehend genannten Materialien, die in Kombination mit dem eigentlichen Lichtemitter eingesetzt werden, einen wesentlichen Einfluss unter anderem auf die Effizienz sowie die Lebensdauer der OLEDs.

Im Stand der Technik werden zahlreiche verschiedene Materialien für den Einsatz in den verschiedenen Schichten von OLEDs vorgeschlagen.

DE 103 30 761 A1 betrifft Mischungen von organischen zur Emission befähigten Halbleitern und Matrixmaterialien, deren Verwendung und Elektronikbauteile, die diese enthalten. Die Mischungen gemäß DE 103 30 761 sind aus mindestens zwei Substanzen aufgebaut, wobei die eine als Matrixmaterial dient und die andere ein zur Emission befähigtes Emissionsmaterial ist. Bei dem Matrixmaterial handelt es sich um eine Verbindung, die mindestens eine Struktureinheit der Form L=X und/oder M=X enthält, wobei der Rest X mindestens ein nicht-bindendes Elektronenpaar aufweist, der Rest L für P, As, Sb oder Bi steht, der Rest M für S, Se, Te steht und gegebenenfalls auch glasartige Schichten bilden kann. In DE 103 30 761 A1 werden zahlreiche verschiedene, geeignete Matrixmaterialien, die unter eine der vorstehend genannten Formeln fallen, genannt. Unter anderem kann es sich bei dem Matrixmaterial um ein cyclisches Phosphazen handeln, wobei das cyclische Phosphazen 4 bis 14 Ringatome aufweisen kann und mit zahlreichen verschiedenen Substituenten am Phosphor substituiert sein kann. Konkrete Beispiele für als Matrixmaterialien geeignete Phosphazene sind in DE 103 30 761 A1 nicht genannt, da gemäß DE 103 30 761 A1 bevorzugt spezielle Spiroverbindungen (keine Phosphazene) als Matrixmaterialien genannt sind.

In JP 08-283416 sind cyclische Phosphazen-Verbindungen sowie organische Dünnfilmelemente genannt, die diese enthalten. Dabei handelt es sich um spezielle cyclische Phosphazene, die 6 oder 8 Ringglieder aufweisen, und worin das Phosphoratom mit Phenoxy-Gruppen oder N,N'-Diphenyl-N-(3-methylphenyl)-1,1'-biphenyl-4,4'-diamino]-3-phenyloxy-Gruppen substituiert ist, wobei mindestens zwei der Reste am Phosphor Phenoxy-Gruppen sind. Gemäß JP 08-283416 zeichnen sich die speziellen Phosphazene dadurch aus, dass sie eine hohe Hitzebeständigkeit und eine hohe amorphe Stabilität aufweisen und frei von Chlorresten und anderen Verunreinigungen sind. Die speziellen cyclischen Phosphazene werden gemäß JP 08-283416 A in Lochtransport- und Lochinjektionsschichten eingesetzt.

JP 2006-278549 A betrifft eine organische Leuchtdiode, die eine Schicht aufweist, die eine (Cyclo)Phosphazen-Verbindung enthält. Als geeignete (Cyclo)Phosphazen-Verbindungen sind in JP 2006-278549 A (Cyclo)Phosphazen-Verbindungen genannt, die 4 bis 8 Ringglieder aufweisen. Geeignete Substituenten am Phosphor sind über Sauerstoff verknüpfte Gruppen -OR¹ bzw. -OR², wobei explizit ein 6-gliedriges Phosphazen mit Substituenten -O-C₆H₄-CF₃ und/oder -O-C₆H₄-F genannt ist.

In V. Vicente et al. (New. J. Chem., 2004, 28, 418-424) sind die Synthesen und Strukturen von Cyclotriphosphazenen mit über Kohlenstoffatome gebundenen heterocyclischen Substituenten offenbart. Bei den eingesetzten heterocyclischen Substituenten handelt es sich um 2-Thienyl-, 3-Thienyl- und 3,3'-Bithienyl-2,2'-ylen-Gruppen. Aufgrund der geometrischen Eigenschaften der genannten Cyclotriphosphazene wird vermutet, dass diese durch Koordination der freien Heteroatome mit Übergangsmetallen gestapelte Strukturen mit möglicherweise interessanten elektronischen Eigenschaften ausbilden können. Konkrete Einsatzmöglichkeiten der Cyclotriphosphazene werden in V. Vincente et al. nicht erwähnt.

In V. Vicente et al. Magn. Res. Chem. 2003, 41:183-192 sind NMR-Untersuchungen von Cyclotriphosphazenen offenbart. Unter anderem werden Strukturen von Cyclotriphosphazenen mit über Kohlenstoffatome gebundenen heterocyclischen Substituenten NMR-spektroskopisch untersucht. Neben den in dem vorstehend genannten Dokument offenbarten Cyclotriphosphazenen, die mit 2-Thienyl-, 3-Thienyl- und 3,3'-Bithienyl-2,2'-ylen-Gruppen substituiert sind, sind Cyclotriphosphazene, die 2-Pyridylgruppen tragen, offenbart, sowie Spirogruppen tragende Cyclotriphosphazene. Die NMR-Untersuchungen sollen der Aufklärung von elektronischen Eigenschaften der untersuchten Cyclotriphosphazene dienen. Konkrete Einsatzmöglichkeiten der Cyclotriphosphazene werden in V. Vincente et al. nicht erwähnt

C. Combes-Chamalet et al. J. Chem. Soc., Perkin Trans. 2, 1997, 15-18 betrifft die Synthese und Struktur von Spiro-Cyclotriphosphazenen. Diese könnten - wie Cyclotriphosphazene- zur Ausbildung von Einschluss-Addukten mit Wasser oder organischen Lösungsmitteln von Interesse sein. Konkrete Einsatzmöglichkeiten der Spirocyclotriphosphazene werden in C. Combes-Chamalet et al. nicht erwähnt.

H. J. Bolink, E. Barea, R. D. Costa, E. Coronado, S. Sudhakar, C. Zhen, A. Sellinger, Organic Electronics 9 (2008) 155-163, beschreiben die Verwendung phosphoreszierender Dendrimere basierend auf Phosphazenen in organischen Leuchtdioden.

Aufgabe der vorliegenden Erfindung gegenüber dem vorstehend genannten Stand der Technik ist die Bereitstellung von für den Einsatz in OLEDs geeigneten Materialien, insbesondere für den Einsatz als Matrixmaterialien in einer Licht-emittierenden Schicht, und/oder für den Einsatz als Lochblockermaterialien, wobei die Licht-emittierende Schicht bevorzugt mindestens einen Blauemitter enthält. Somit sollen insbesondere Komplementärmaterialien für OLEDs (bevorzugt Matrixmaterialien, Ladungstransportmaterialien, Blockermaterialien, Ladungsinjektionsmaterialien) bereitgestellt werden, die eine hohe Triplett-Energie aufweisen, damit eine Lichtemission des in den OLEDs eingesetzten Blauemitters gewährleistet ist. Des Weiteren sollen die Materialien zur Bereitstellung von OLEDs geeignet sein, die gute Effizienzen, gute operative Lebensdauern sowie eine hohe Stabilität gegenüber Temperaturbelastung und eine niedrige Einsatz- und Betriebsspannung der OLEDs gewährleisten.

Diese Aufgabe wird gelöst durch eine organische Leuchtdiode enthaltend mindestens eine cyclische Phosphazenverbindung der allgemeinen Formel (I) 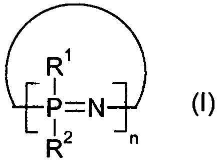 worin bedeuten:
- n: 3 bis 8, bevorzugt 3 oder 4, besonders bevorzugt 3;
- R¹, R²: unabhängig voneinander substituiertes oder unsubstituiertes Aryl, substituiertes oder unsubstituiertes Heteroaryl, substituiertes oder unsubstituiertes Alkyl, substituiertes oder unsubstituiertes Aryloxy, substituiertes oder unsubstituiertes Alkyloxy, unsubstituiertes oder substituiertes Cycloalkyl, unsubstituiertes oder substituiertes Heterocycloalkyl, unsubstituiertes oder substituiertes Alkenyl, unsubstituiertes oder substituiertes Alkinyl, unsubstituiertes oder substituiertes Aralkyl oder Halogen, bevorzugt unabhängig voneinander substituiertes oder unsubstituiertes Aryl, substituiertes oder unsubstituiertes Heteroaryl, substituiertes oder unsubstituiertes Alkyl, substituiertes oder unsubstituiertes Aryloxy, substituiertes oder unsubstituiertes Alkyloxy, besonders bevorzugt unabhängig voneinander substituiertes oder unsubstituiertes Aryl, substituiertes oder unsubstituiertes Heteroaryl oder substituiertes oder unsubstituiertes Aryloxy, ganz besonders bevorzugt unabhängig voneinander substituiertes oder unsubstituiertes Aryl oder substituiertes oder unsubstituiertes Heteroaryl, wobei R¹ und R² in den n Gruppen jeweils gleich oder verschieden sein können;
oder
die vorstehend genannten Reste R¹ und R² sind unabhängig voneinander in den n Gruppen der allgemeinen Formel (I) über eine Bindung, eine C₁-C₆-Alkylenbrücke, bevorzugt eine C₁-C₂-Alkylenbrücke, eine Arylenbrücke, bevorzugt eine 1,2-Phenylenbrücke, oder eine C₂-C₆-Alkenylenbrücke, bevorzugt eine C₂-C₄-Alkenylenbrücke, miteinander verknüpft, so dass die Reste R¹ und R² gemeinsam mit dem P-Atom, mit dem sie verknüpft sind, einen Cyclus bilden; bevorzugt bilden R¹ und R² - in dem Fall, wenn sie miteinander verknüpft sind - gemeinsam unabhängig voneinander in den n Gruppen der allgemeinen Formel (I) eine der folgenden Gruppen: 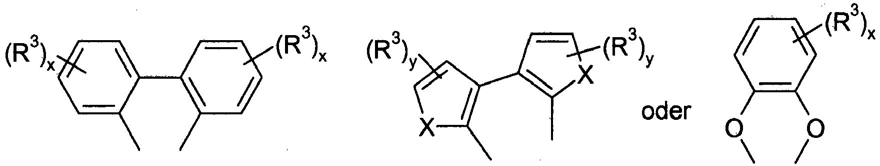 worin bedeuten:
- R³: unabhängig voneinander substituiertes oder unsubstituiertes Aryl, substituiertes oder unsubstituiertes Heteroaryl, substituiertes oder unsubstituiertes Alkyl, substituiertes oder unsubstituiertes Aryloxy, substituiertes oder unsubstituiertes Alkyloxy, unsubstituiertes oder substituiertes Cycloalkyl, unsubstituiertes oder substituiertes Heterocycloalkyl, unsubstituiertes oder substituiertes Alkenyl, unsubstituiertes oder substituiertes Alkinyl, unsubstituiertes oder substituiertes Aralkyl oder Halogen, bevorzugt F oder Pseudohalogen, bevorzugt CN, bevorzugt unabhängig voneinander substituiertes oder unsubstituiertes Aryl, substituiertes oder unsubstituiertes Heteroaryl, substituiertes oder unsubstituiertes Alkyl, substituiertes oder unsubstituiertes Aryloxy, substituiertes oder unsubstituiertes Alkyloxy oder F oder CN;
- x: unabhängig voneinander 0, 1, 2, 3 oder 4, bevorzugt 0, 1 oder 2;
- y: unabhängig voneinander 0, 1 oder 2, bevorzugt 0 oder 1.

Die vorliegende Erfindung ist dann dadurch gekennzeichet, dass mindestens einer der in den n Gruppen vorliegenden Reste R¹ oder R² mit mindestens einem der P-Atome der cyclischen Phosphazene der Formel (I) nicht über ein Sauerstoffatom verknüpft ist, wobei der mindestens eine Rest R¹ oder R² bevorzugt über ein Kohlenstoffatom verknüpft ist.

Die in der erfindungsgemäßen OLED eingesetzten cyclischen Phosphazenverbindungen zeichnen sich durch eine hervorragende Eignung für den Einsatz in OLEDs aus. Insbesondere weisen die erfindungsgemäß eingesetzten cyclischen Phosphazenverbindungen, worin mindestens einer der Reste R¹ oder R² mit mindestens einem der P-Atome nicht über ein Sauerstoffatom verknüpft ist und bevorzugt über ein Kohlenstoffatom verknüpft ist, gegenüber entsprechenden cyclischen Phosphazenen, deren Substituenten ausschließlich über Sauerstoffatome mit den Phosphoratomen verknüpft sind, dadurch aus, dass sie im Allgemeinen hohe Glasübergangstemperaturen und in dünnen Schichten, wie sie in OLEDs eingesetzt werden, eine höhere Amorphizität aufweisen. Dadurch wird eine optimale Performance von OLEDs, die mindestens eine Verbindung der Formel (I) enthalten, erreicht. Des Weiteren weisen die erfindungsgemäß eingesetzten cyclischen Phosphazenverbindungen bevorzugt einen Energieunterschied zwischen dem elektronisch angeregten Triplett-Zustand T₁ und dem Grundzustand S₀ auf, der ausreichend groß ist, damit sie für den Einsatz als Komplementärmaterialien, insbesondere Matrixmaterialien, Blockermaterialien, Ladungstransportmaterialien und/oder Ladungsinjektionsmaterialien für Blauemitter in Frage kommen.

In Abhängigkeit von ihrem Substitutionsmuster und den elektronischen Eigenschaften der in der OLED verwendeten Schichten können die erfindungsgemäß verwendeten cyclischen Phosphazenverbindungen neben dem Einsatz als Matrixmaterialien in der Licht-emittierenden Schicht und dem Einsatz als Loch-/Excitonenblockermaterialien auch als Elektronenleiter, Elektroneninjektionsmaterialien und/oder Elektronen-/Excitonenblockermaterialien, Loch-Injektionsmaterialien und/oder Lochleiter eingesetzt werden. Entsprechende Schichten von OLEDs sind dem Fachmann grundsätzlich bekannt und zum Beispiel in WO 2005/113704 oder WO 2005/019373 genannt.

Bevorzugt werden die erfindungsgemäß eingesetzten cyclischen Phosphazenverbindungen als Matrixmaterial in der Licht-emittierenden Schicht und/oder als Loch-/Excitonenblockermaterial und/oder Elektronenleiter, besonders bevorzugt als Matrixmaterial in der Licht-emittierenden Schicht und/oder Loch-/Excitonenblocker, eingesetzt.

Im Sinne der vorliegenden Anmeldung haben die Begriffe unsubstituierter oder substituierter Arylrest oder -gruppe, unsubstituierter oder substituierter Heteroarylrest oder-gruppe, unsubstituierter oder substituierter Alkylrest oder -gruppe, unsubstituierter oder substituierter Cycloalkylrest oder -gruppe, unsubstituierter oder substituierter Heterocycloalkylrest oder -gruppe, unsubstituierter oder substituierter Alkenylrest oder - gruppe, unsubstituierter oder substituierter Alkinylrest oder -gruppe, unsubstituierter oder substituierter Aralkylrest oder -gruppe, und Gruppen mit Donor- und/oder Akzeptorwirkung die folgenden Bedeutungen:

Unter einem Arylrest (oder -gruppe) ist ein Rest mit einem Grundgerüst von 6 bis 30 Kohlenstoffatomen, bevorzugt 6 bis 18 Kohlenstoffatomen zu verstehen, der aus einem aromatischen Ring oder mehreren kondensierten aromatischen Ringen aufgebaut ist. Geeignete Grundgerüste sind zum Beispiel Phenyl, Naphthyl, Anthracenyl oder Phenanthrenyl. Dieses Grundgerüst kann unsubstituiert sein (d. h., dass alle Kohlenstoffatome, die substituierbar sind, Wasserstoffatome tragen), oder an einer, mehreren oder allen substituierbaren Positionen des Grundgerüsts substituiert sein. Geeignete Substituenten sind zum Beispiel Deuterium, Alkoxyreste, Aryloxyreste, Alkylaminogruppen, Arylaminogruppen, Carbazoylgruppen, Silylgruppen, Alkylreste, bevorzugt Alkylreste mit 1 bis 8 Kohlenstoffatomen, besonders bevorzugt Methyl, Ethyl oder i-Propyl, Arylreste, bevorzugt C₆-Arylreste, die wiederum substituiert oder unsubstituiert sein können, Heteroarylreste, bevorzugt Heteroarylreste, die mindestens ein Stickstoffatom enthalten, besonders bevorzugt Pyridylreste, Alkenylreste, bevorzugt Alkenylreste, die eine Doppelbindung tragen, besonders bevorzugt Alkenylreste mit einer Doppelbindung und 1 bis 8 Kohlenstoffatomen, oder Gruppen mit Donor- oder Akzeptorwirkung. Geeignete Gruppen, mit Donor- oder Akzeptorwirkung sind nachstehend genannt. Ganz besonders bevorzugt tragen die substituierten Arylreste Substituenten ausgewählt aus der Gruppe bestehend aus Methyl, Ethyl, Isopropyl, Alkoxy, Heteroaryl, CN und Aryloxy. Bevorzugt ist der Arylrest oder die Arylgruppe ein C₆-C₁₈-Arylrest, besonders bevorzugt ein C₆-Arylrest, der gegebenenfalls mit mindestens einem oder mehreren der vorstehend genannten Substituenten substituiert ist. Besonders bevorzugt weist der C₆-C₁₈-Arylrest, bevorzugt C₆-Arylrest, keinen, einen, zwei, drei oder vier, ganz besonders bevorzugt keinen, einen oder zwei der vorstehend genannten Substituenten auf. Besonders bevorzugte als Reste R¹ und R² geeignete Arylreste sind in einer Ausführungsform C₆-Arylreste, die einen oder zwei Substituenten aufweisen.

Unter einem Heteroarylrest oder einer Heteroarylgruppe sind Reste zu verstehen, die sich von den vorstehend genannten Arylresten dadurch unterscheiden, dass in dem Grundgerüst der Arylreste mindestens ein Kohlenstoffatom durch ein Heteroatom ersetzt ist, sowie dadurch, dass das Grundgerüst der Heteroarylreste bevorzugt 5 bis 18 Ringatome aufweist. Bevorzugte Heteroatome sind N, O und S. Besonders bevorzugte als Reste R¹ und R² geeignete Heteroarylreste sind stickstoffhaltige Heteroarylreste. Ganz besonders bevorzugt sind ein oder zwei Kohlenstoffatome des Grundgerüsts durch Heteroatome, bevorzugt Stickstoff, ersetzt. Insbesondere bevorzugt ist das Grundgerüst ausgewählt aus Systemen wie Pyridin, Pyrimidin und fünfgliedrigen Heteroaromaten wie Pyrrol, Furan, Pyrazol, Imidazol, Thiophen, Oxazol, Thiazol, Triazol. Des Weiteren kann es sich bei den Heteroarylresten um kondensierte Ringsysteme handeln, z. B. um Carbazolylreste oder Azacarbazoylreste. Das Grundgerüst kann an einer, mehreren oder allen substituierbaren Positionen des Grundgerüsts substituiert sein. Geeignete Substituenten sind dieselben, die bereits bezüglich der Arylgruppen genannt wurden.

Unter einem Alkylrest oder einer Alkylgruppe ist ein Rest mit 1 bis 20 Kohlenstoffatomen, bevorzugt 1 bis 10 Kohlenstoffatomen, besonders bevorzugt 1 bis 8, ganz besonders bevorzugt 1 bis 4 Kohlenstoffatomen zu verstehen. Dieser Alkylrest kann verzweigt oder unverzweigt sein und gegebenenfalls mit einem oder mehreren Heteroatomen, bevorzugt Si, N, O oder S, besonders bevorzugt N, O oder S, unterbrochen sein. Des Weiteren kann dieser Alkylrest mit einem oder mehreren der bezüglich der Arylgruppen genannten Substituenten substituiert sein. Es ist ebenfalls möglich, dass der Alkylrest eine oder mehrere (Hetero-)-Arylgruppen trägt. Im Sinne der vorliegenden Anmeldung stellen z. B. Benzylreste somit substituierte Alkylreste dar. Dabei sind alle der vorstehend aufgeführten (Hetero-)Arylgruppen geeignet. Besonders bevorzugt sind die Alkylreste ausgewählt aus der Gruppe bestehend aus Methyl, Ethyl, iso-Propyl, n-Propyl, n-Butyl, iso-Butyl und tert-Butyl, ganz besonders bevorzugt sind Methyl und Ethyl.

Unter einem Cycloalkylrest oder einer Cycloalkylgruppe ist ein Rest mit 3 bis 20 Kohlenstoffatomen, bevorzugt 3 bis 10 Kohlenstoffatomen, besonders bevorzugt 3 bis 8 Kohlenstoffatomen zu verstehen. Dieses Grundgerüst kann unsubstituiert sein (d. h., dass alle Kohlenstoffatome, die substituierbar sind, Wasserstoffatome tragen), oder an einer, mehreren oder allen substituierbaren Positionen des Grundgerüsts substituiert sein. Geeignete Substituenten sind die bereits vorstehend bezüglich der Arylreste genannten Gruppen. Beispiele für geeignete Cycloalkylreste sind Cyclopropyl, Cyclopentyl und Cyclohexyl.

Unter einem Heterocycloalkylrest oder einer Heterocycloalkylgruppe sind Reste zu verstehen, die sich von den vorstehend genannten Cycloalkylresten dadurch unterscheiden, dass in dem Grundgerüst der Cycloalkylreste mindestens ein Kohlenstoffatom durch ein Heteroatom ersetzt ist. Bevorzugte Heteroatome sind N, O und S. Ganz besonders bevorzugt sind ein oder zwei Kohlenstoffatome des Grundgerüsts der Cycloalkylreste durch Heteroatome ersetzt. Beispiele für geeignete Heterocycloalkylreste sind Reste abgeleitet von Pyrrolidin, Piperidin, Piperazin, Tetrahydrofuran, Dioxan.

Unter einem Alkenylrest oder einer Alkenylgruppe ist ein Rest zu verstehen, der den vorstehend genannten Alkylresten mit mindestens zwei Kohlenstoffatomen entspricht, mit dem Unterschied, dass mindestens eine C-C-Einfachbindung des Alkylrests durch eine C-C-Doppelbindung ersetzt ist. Bevorzugt weist der Alkenylrest eine oder zwei Doppelbindungen auf.

Unter einem Alkinylrest oder einer Alkinylgruppe ist ein Rest zu verstehen, der den vorstehend genannten Alkylresten mit mindestens zwei Kohlenstoffatomen entspricht, mit dem Unterschied, dass mindestens eine C-C-Einfachbindung des Alkylrests durch eine C-C-Dreifachbindung ersetzt ist. Bevorzugt weist der Alkinylrest eine oder zwei Dreifachbindungen auf.

Unter einer C₁-C₆-Alkylenbrücke, bevorzugt einer C₁-C₂-Alkylenbrücke, ist eine Alkylengruppe mit 1 bis 6, bevorzugt 1 oder 2, Kohlenstoffatomen in der Hauptkette zu verstehen. Die Alkylenbrücke kann ggf. mit einem oder mehreren der bezüglich der Arylgruppen genannten Substituenten substituiert sein. Es ist ebenfalls möglich, dass der Alkylenrest eine oder mehrere (Hetero-)-Arylgruppen trägt. Des Weiteren können in der Hauptkette der Alkylenbrücke ein oder mehrere Heteroatome, bevorzugt -O- oder -NR'-, wobei R' ein Alkyl-, Aryl-, Heteroaryl-, Cycloalkyl- oder Heterocycloalkylrest ist, vorliegen.

Unter einer C₂-C₆-Alkenylenbrücke, bevorzugt einer C₂-C₄-Alkenylenbrücke, ist eine Alkenylengruppe mit 2 bis 6, bevorzugt 2 bis 4, Kohlenstoffatomen in der Hauptkette zu verstehen. Die Alkenylenbrücke kann ggf. mit einem oder mehreren der bezüglich der Arylgruppen genannten Substituenten substituiert sein. Es ist ebenfalls möglich, dass der Alkenylenrest eine oder mehrere (Hetero-)-Arylgruppen trägt. Des Weiteren können in der Hauptkette der Alkenylenbrücke ein oder mehrere Heteroatome, bevorzugt -Ooder-NR'-, wobei R' ein Alkyl-, Aryl-, Heteroaryl-, Cycloalkyl- oder Heterocycloalkylrest ist, vorliegen.

Unter einer Arylenbrücke ist eine Arylengruppe zu verstehen, die bevorzugt 6 bis 18 Kohlenstoffatome im Grundgerüst aufweist. Die Arylengruppe kann aus einem aromatischen Ring oder mehreren kondensierten aromatischen Ringen aufgebaut sein. Geeignete Grundgerüste sind zum Beispiel Phenylen, Naphthylen, Anthracenylen oder Phenanthrenylen. Die Arylenbrücke kann ggf. mit einem oder mehreren der bezüglich der Arylgruppen genannten Substituenten substituiert sein. Bevorzugt handelt es sich bei der Arylenbrücke um eine Phenylenbrücke, die besonders bevorzugt unsubstituiert ist. Ganz besonders bevorzugt ist die Phenylenbrücke eine 1,2-Phenylenbrücke.

Unter Halogengruppen sind bevorzugt F, Cl und Br, besonders bevorzugt F und Cl, ganz besonders bevorzugt F, zu verstehen.

Unter Pseudohalogengruppen sind bevorzugt CN, SCN und OCN, besonders bevorzugt CN, zu verstehen.

Unter einer Gruppe oder einem Substituenten mit Donor- oder Akzeptorwirkung sind im Sinne der vorliegenden Anmeldung die folgenden Gruppen zu verstehen:

Unter Gruppen mit Donorwirkung sind Gruppen zu verstehen, die einen +I- und/oder +M-Effekt aufweisen, und unter Gruppen mit Akzeptorwirkung sind Gruppen zu verstehen, die einen -I- und/oder -M-Effekt aufweisen. Geeignete Gruppen, mit Donor- oder Akzeptorwirkung sind Halogenreste, bevorzugt F, Cl, Br, Alkoxyreste oder Aryloxyreste, OR², Carbonylreste, Esterreste, sowohl Oxycarbonyl als auch Carbonyloxy, Aminogruppen, NR², Amidreste, CH₂F-Gruppen, CHF₂-Gruppen, CF₃-Gruppen, CN-Gruppen, Thiogruppen, Sulfonsäuregruppen, Sulfonsäureestergruppen, Boronsäuregruppen, Boronsäureestergruppen, Phosphonsäuregruppen, Phosphonsäureestergruppen, Phosphinreste, Sulfoxidreste, Sulfonylreste, Sulfidreste, SR², Nitrogruppen, OCN, Boranreste, Silylgruppen, Stannatreste, Iminogruppen, Hydrazinreste, Hydrazonreste, Oximreste, Nitrosogruppen, Diazogruppen, Phosphinoxidgruppen, Hydroxygruppen oder SCN-Gruppen. Ganz besonders bevorzugt sind F,,CN, Aryloxy, Alkoxy, Amino, CF₃-Gruppen, Sulfonyl, Silyl, Sulfid und Heteroaryl. Insbesondere ganz besonders bevorzugt sind Heteroaryl, Silyl, Alkoxy oder Aryloxy, Amino und CN.

Die vorstehend genannten Gruppen mit Donor- oder Akzeptorwirkung schließen nicht aus, dass weitere der in der vorliegenden Anmeldung genannte Reste und Substituenten, die nicht in der vorstehenden Liste der Gruppen mit Donor- oder Akzeptorwirkung aufgeführt sind, eine Donor- oder Akzeptorwirkung aufweisen.

Die Arylreste oder -gruppen, Heteroarylreste oder -gruppen, Alkylreste oder -gruppen, Cycloalkylreste oder -gruppen, Heterocycloalkylreste oder -gruppen, Alkenylreste oder -gruppen, Alkinylreste oder -gruppen und Gruppen mit Donor- und/oder Akzeptorwirkung, sowie die Alkylen- und Arylenreste oder -gruppen können - wie vorstehend erwähnt - substituiert oder unsubstituiert sein. Unter einer unsubstituierten Gruppe ist im Sinne der vorliegenden Anmeldung eine Gruppe zu verstehen, worin die substituierbaren Atome der Gruppe Wasserstoffatome tragen. Unter einer substituierten Gruppe ist im Sinne der vorliegenden Anmeldung eine Gruppe zu verstehen, worin ein oder mehrere substituierbare Atom(e) mindestens an einer Position anstelle eines Wasserstoffatoms einen Substituenten tragen. Geeignete Substituenten sind die vorstehend bezüglich der Arylreste oder -gruppen genannten Substituenten.

Kommen Reste mit denselben Nummerierungen mehrfach in den Verbindungen gemäß der vorliegenden Anmeldung vor, so können diese Reste jeweils unabhängig voneinander die genannten Bedeutungen aufweisen.

Bei dem mindestens einen in den n-Gruppen der cyclischen Phosphazenverbindungen der allgemeinen Formel (I) vorliegenden Rest R¹ oder R², der mit mindestens einem der P-Atome der cyclischen Phosphazenverbindungen nicht über ein Sauerstoffatom und bevorzugt über ein Kohlenstoffatom verknüpft ist, handelt es sich besonders bevorzugt um einen unsubstituierten oder substituierten Arylrest oder um einen unsubstituierten oder substituierten Heteroarylrest. Geeignete Arylreste und Heteroarylreste sowie geeignete Substituenten sind vorstehend genannt. Ganz besonders bevorzugt sind C₆-Arylreste, z. B. unsubstituierte C₆-Arylreste oder mit einem oder zwei Alkyloder Alkoxy-Gruppen substituierte C₆-Arylreste. Bei dem mindestens einen Rest R¹ oder R² kann es sich z. B. um Phenyl, 3,5-Dimethylphenyl oder 4-Methoxyphenyl handeln. Besonders bevorzugte Heteroarylreste sind Heteroarylreste, die 5 bis 18 Ringatome, ganz besonders bevorzugt 5 bis 13 Ringatome aufweisen und substituiert oder unsubstituiert sein können. Ganz besonders bevorzugt sind stickstoffhaltige Heteroarylreste. Grundsätzlich können die stickstoffhaltigen Heteroarylreste in den erfindungsgemäß eingesetzten cyclischen Phosphazenverbindungen der allgemeinen Formel (I) über ein Stickstoffatom oder ein Kohlenstoffatom mit mindestens einem der P-Atome der cyclischen Phosphazenverbindungen verknüpft sein. Bevorzugt sind die stickstoffhaltigen Heteroarylreste über ein Kohlenstoffatom mit dem P-Atom verknüpft. Beispiele für geeignete Heteroarylreste sind 3-Pyridyl, 4-Pyridyl, Azacarbazolyl oder 3-Carbazolyl.

Des Weiteren können die Reste R¹ und R² in einer oder mehreren der n Gruppen der cyclischen Phosphazenverbindungen der allgemeinen Formel (I) unabhängig voneinander über eine Bindung, eine C₁-C₆-Alkylenbrücke, bevorzugt eine C₁-C₂-Alkylenbrücke, eine Arylenbrücke, bevorzugt eine 1,2-Phenylenbrücke, oder eine C₂-C₆-Alkenylenbrücke, bevorzugt eine eine C₂-C₄-Alkenylenbrücke, miteinander verknüpft sein, so dass die Reste R¹ und R² gemeinsam mit dem P-Atom, mit dem sie verknüpft sind, einen Cyclus bilden; bevorzugt bilden R¹ und R² - in dem Fall, wenn sie miteinander verknüpft sind - gemeinsam unabhängig voneinander in den n Gruppen der allgemeinen Formel (I) eine der folgenden Gruppen: 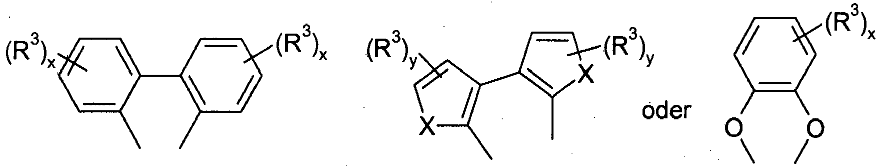 worin bedeuten:
- R³: unabhängig voneinander substituiertes oder unsubstituiertes Aryl, substituiertes oder unsubstituiertes Heteroaryl, substituiertes oder unsubstituiertes Alkyl, substituiertes oder unsubstituiertes Aryloxy, substituiertes oder unsubstituiertes Alkyloxy, unsubstituiertes oder substituiertes Cycloalkyl, unsubstituiertes oder substituiertes Heterocycloalkyl, unsubstituiertes oder substituiertes Alkenyl, unsubstituiertes oder substituiertes Alkinyl, unsubstituiertes oder substituiertes Aralkyl oder Halogen, bevorzugt F oder Pseudohalogen, bevorzugt CN, bevorzugt unabhängig voneinander substituiertes oder unsubstituiertes Aryl, substituiertes oder unsubstituiertes Heteroaryl, substituiertes oder unsubstituiertes Alkyl, substituiertes oder unsubstituiertes Aryloxy, substituiertes oder unsubstituiertes Alkyloxy oder F oder CN;
- x: unabhängig voneinander 0, 1, 2, 3 oder 4, bevorzugt 0, 1 oder 2;
- y: unabhängig voneinander 0, 1 oder 2, bevorzugt 0 oder 1.

Ganz besonders bevorzugt sind solche cyclischen Phosphazenverbindungen der allgemeinen Formel (I), worin bedeuten:
- R¹, R²: unabhängig voneinander substituiertes oder unsubstituiertes Aryl oder substituiertes oder unsubstituiertes Heteroaryl, wobei R¹ und R² in den n-Gruppen jeweils gleich oder verschieden sein können.

Bevorzugt sind R¹ und R² in den n-Gruppen jeweils gleich. Geeignete substituierte oder unsubstituierte Arylreste sowie geeignete substituierte oder unsubstituierte Heteroarylreste sind vorstehend genannt. Besonders geeignete substituierte oder unsubstituierte Arylreste sowie besonders geeignete substituierte oder unsubstituierte Heteroarylreste sind die bezüglich dem mindestens einen in den n-Gruppen vorliegenden Rest R¹ oder R², der mit mindestens einem der P-Atome der cyclischen Phosphazenverbindungen nicht über ein Sauerstoffatom und bevorzugt über ein Kohlenstoffatom verknüpft ist, genannten Reste. Das bedeutet, in einer ganz besonders bevorzugten Ausführungsform sind alle Reste R¹ und R² der cyclischen Phosphazenverbindungen der Formel (I) nicht über ein Sauerstoffatom, sondern bevorzugt über ein Stickstoff- oder Kohlenstoffatom und besonders bevorzugt über ein Kohlenstoffatom mit den jeweiligen P-Atomen verknüpft.

In einer weiteren bevorzugten Ausführungsform ist n in den cyclischen Phosphazenen der allgemeinen Formel (I) 3 oder 4, besonders bevorzugt 3. Das bedeutet, dass cyclische Phosphazenverbindungen der Formel (I), die 6 oder 8 Ringglieder aufweisen, besonders bevorzugt sind, wobei cyclische Phosphazenverbindungen der Formel (I), die 6 Ringglieder aufweisen (Cyclotriphosphazene), ganz besonders bevorzugt sind.

Weiter ganz besonders bevorzugt sind cyclische Phosphazenverbindungen der Formel (I), worin n 3 bedeutet und der mindestens eine in den n-Gruppen vorliegende Rest R¹ oder R², der mit mindestens einem der P-Atome nicht über ein Sauerstoffatom und bevorzugt über ein Kohlenstoffatom verknüpft ist, ein substituierter oder unsubstituierter Arylrest oder ein substituierter oder unsubstituierter Heteroarylrest ist.

Ganz besonders bevorzugt weisen die erfindungsgemäß eingesetzten cyclischen Phosphazenverbindungen somit die Formel (Ia) auf 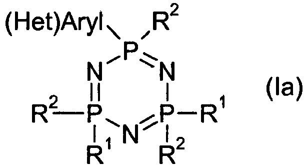 worin bedeuten
- (Het)Aryl: unsubstituiertes oder substituiertes C₆₋₁₄-Aryl, z. B. Phenyl, C₁-C₆-Alkylphenyl, C₁-C₆-Dialkylphenyl, C₁-C₆-Alkoxyphenyl oder C₁-C₆-Dialkoxyphenl, oder unsubstituiertes oder substituiertes Heteroaryl, das 5 bis 14 Ringatome aufweist und über ein Kohlenstoffatom mit dem P des Phosphazenrings verknüpft ist, z. B. unsubstituiertes oder substituiertes Carbazolyl, unsubstituiertes oder substituiertes Azacarbazolyl oder unsubstituiertes oder substituiertes Pyridyl;
- R¹, R²: unabhängig voneinander substituiertes oder unsubstituiertes Aryl, substituiertes oder unsubstituiertes Heteroaryl, substituiertes oder unsubstituiertes Alkyl, substituiertes oder unsubstituiertes Aryloxy, substituiertes oder unsubstituiertes Alkyloxy, wobei R¹ und R² jeweils gleich oder verschieden sein können, bevorzugt unabhängig voneinander substituiertes oder unsubstituiertes Aryl oder substituiertes oder unsubstituiertes Heteroaryl, wobei R¹ und R² jeweils gleich oder verschieden sein können; besonders bevorzugt unsubstituiertes oder substituiertes C₆₋₁₄-Aryl, z. B. Phenyl, C₁-C₆-Alkylphenyl, C₁-C₆-Dialkylphenyl, C₁-C₆-Alkoxyphenyl oder C₁-C₆-Dialkoxyphenl oder unsubstituiertes oder substituiertes Heteroaryl, das 5 bis 14 Ringatome aufweist, z. B. oder unsubstituiertes oder substituiertes Pyridyl, unsubstituiertes oder substituiertes Carbazolyl, unsubstituiertes oder substituiertes Azacarbazolyl wobei R¹ und R² jeweils gleich oder verschieden sein können;
oder
die vorstehend genannten Reste R¹ und R² sind unabhängig voneinander über eine Bindung, eine C₁-C₆-Alkylenbrücke, bevorzugt eine C₁-C₂-Alkylenbrücke, eine Arylenbrücke, bevorzugt eine 1,2-Phenylenbrücke, oder eine C₂-C₆-Alkenylenbrücke, bevorzugt eine eine C₂-C₄-Alkenylenbrücke, miteinander verknüpft, so dass die Reste R¹ und R² gemeinsam mit dem P-Atom, mit dem sie verknüpft sind, einen Cyclus bilden; bevorzugt bilden R1 und R2 - in dem Fall, wenn sie miteinander verknüpft sind - gemeinsam unabhängig voneinander in den n Gruppen der allgemeinen Formel (I) eine der folgenden Gruppen: 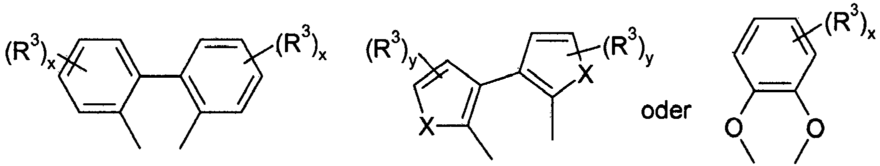
worin bedeuten:
- R³: unabhängig voneinander substituiertes oder unsubstituiertes Aryl, substituiertes oder unsubstituiertes Heteroaryl, substituiertes oder unsubstituiertes Alkyl, substituiertes oder unsubstituiertes Aryloxy, substituiertes oder unsubstituiertes Alkyloxy, unsubstituiertes oder substituiertes Cycloalkyl, unsubstituiertes oder substituiertes Heterocycloalkyl, unsubstituiertes oder substituiertes Alkenyl, unsubstituiertes oder substituiertes Alkinyl, unsubstituiertes oder substituiertes Aralkyl oder Halogen, bevorzugt F oder Pseudohalogen, bevorzugt CN, bevorzugt unabhängig voneinander substituiertes oder unsubstituiertes Aryl, substituiertes oder unsubstituiertes Heteroaryl, substituiertes oder unsubstituiertes Alkyl, substituiertes oder unsubstituiertes Aryloxy, substituiertes oder unsubstituiertes Alkyloxy oder F oder CN;
- x: unabhängig voneinander 0, 1, 2, 3 oder 4, bevorzugt 0, 1 oder 2;
- y: unabhängig voneinander 0, 1 oder 2, bevorzugt 0 oder 1.

Ganz besonders bevorzugte Ausführungsformen für (Het)Aryl sowie für R¹ und R² sind die vorstehend für R¹ und R² genannten Reste.

Ganz besonders bevorzugt sind die Reste R¹ und R² sowie der Rest (Het)Aryl in den cyclischen Phosphazen-Verbindungen der Formel (Ia) identisch, d.h. ganz besonders bevorzugt sind cyclische Phosphazenverbindungen der allgemeinen Formel (Iaa): 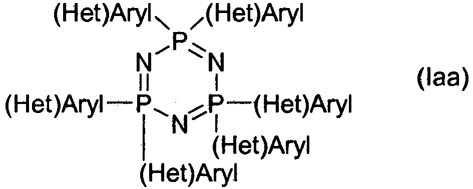 worin
- (Het)Aryl: unsubstituiertes oder substituiertes C₆₋₁₄-Aryl, z. B. Phenyl, C₁-C₆-Alkylphenyl, C₁-C₆-Dialkylphenyl, C₁-C₆-Alkoxyphenyl oder C₁-C₆-Dialkoxyphenyl oder unsubstituiertes oder substituiertes Heteroaryl, das 5 bis 14 Ringatome aufweist und über ein Kohlenstoffatom mit dem P des Phosphazenrings verknüpft ist, z. B. unsubstituiertes oder substituiertes Carbazolyl, unsubstituiertes oder substituiertes Azacarbazolyl oder unsubstituiertes oder substituiertes Pyridyl, bedeutet.

Bevorzugt bedeutet (Het)Aryl unsubstituiertes oder substituiertes C₆-Aryl oder unsubstituiertes oder substituiertes stickstoffhaltiges C₆-Heteroaryl oder substituiertes oder unsubstituiertes Carbazolyl oder substituiertes oder unsubstituiertes Azacarbazolyl. Geeignete Substituenten sind vorstehend genannt. Bevorzugte Substituenten sind C₁- bis C₆-Alkyl, z. B. Methyl, Ethyl, n-Propyl, iso-Propyl, n-Butyl, iso-Butyl, sek.-Butyl, tert.Butyl, CN und C₁- bis C₆-Alkoxy, z. B. Methoxy, Ethoxy. Beispiele für geeignete (Het)Arylreste sind Phenyl, C₁-C₆-Alkylphenyl wie Methylphenyl, C₁-C₆-Dialkylphenyl wie Dimethylphenyl, C₁-C₆-Alkoxyphenyl wie Methoxyphenyl, C₁-C₆-Dialkoxyphenyl wie Dimethoxyphenyl, Pyridyl, C₁-C₆-Alkylpyridyl wie Methylpyridyl, Carbazolyl, C₁-C₆Alkylcarbazolyl wie Methylcarbazolyl, N-C₁-C₆-Alkylcarbazolyl wie N-Methylcarbazolyl, C₁-C₆-Dialkylcarbazolyl wie Dimethylcarbazolyl, Azacarbazolyl und N-C₁-C₆-Azacarbazolyl wie N-Methyl-Azacarbazolyl.

Beispiele für besonders geeignete cyclische Phosphazenverbindungen der Formel (Iaa) sind die nachstehend genannten Phosphazenverbindungen der Formeln Iaaa bis Iaar

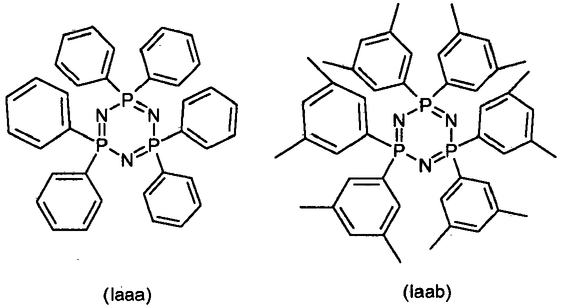

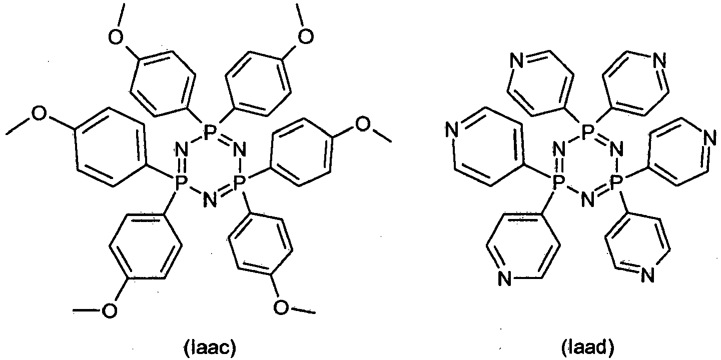

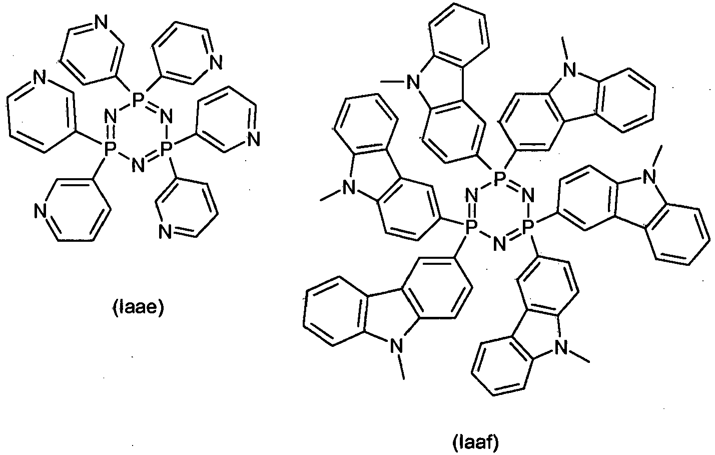

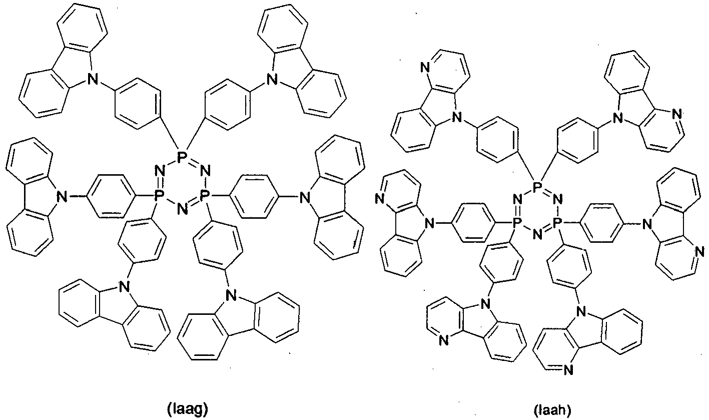

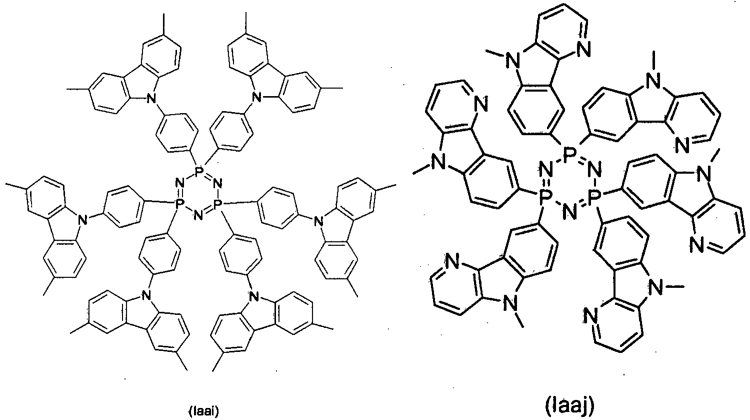

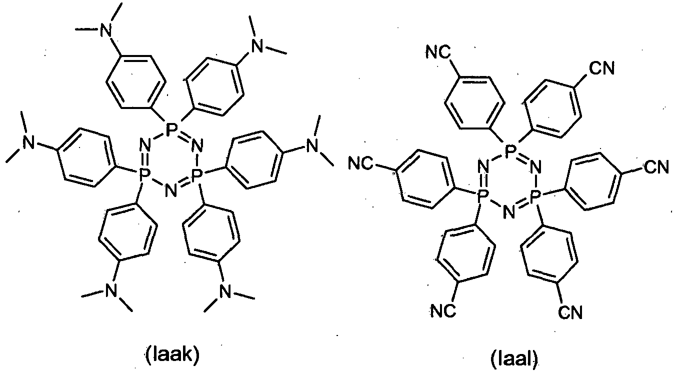

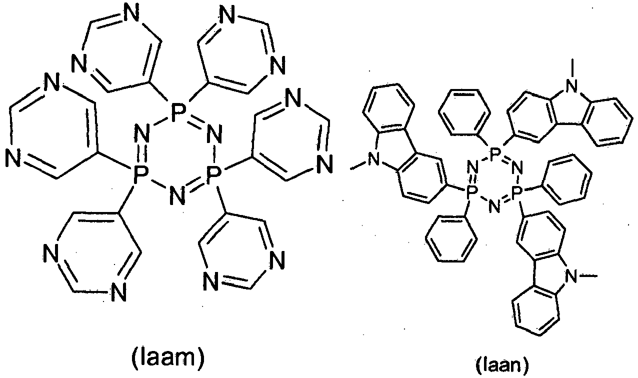

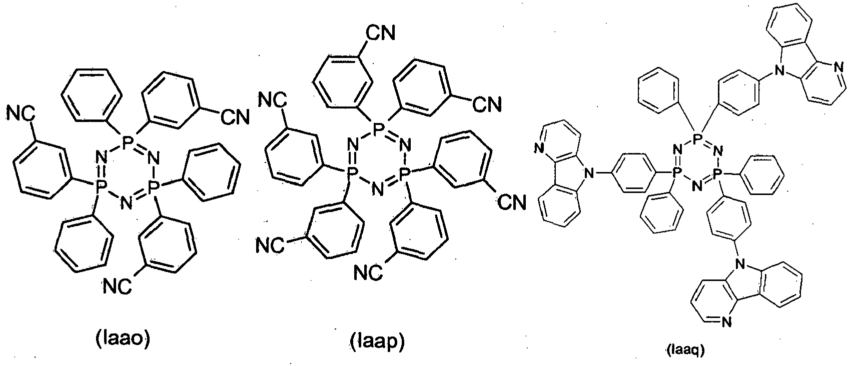

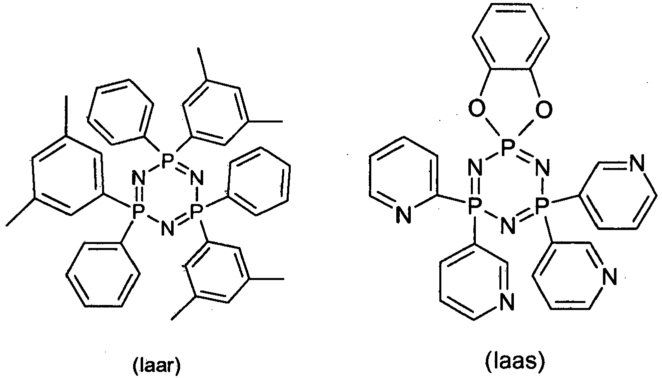

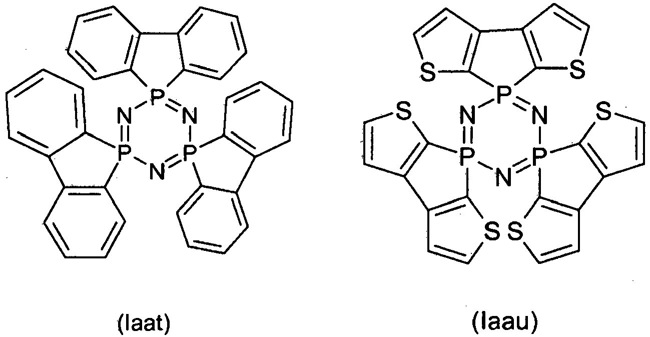

Es wurde gefunden, dass insbesondere die vorstehend genannten cyclischen Phosphazenverbindungen der allgemeinen Formel (Ia), insbesondere die Verbindungen, worin R¹ und R² dieselben Bedeutungen aufweisen wie (Het)Aryl, d. h. worin alle Reste am Phosphazen-Grundgerüst aromatische oder heteroaromatische Reste sind, die über ein Kohlenstoffatom mit dem jeweiligen P-Atom verbunden sind, - neben hohen Glasübergangstemperaturen und einer guten Amorphizität - sich durch einen E-nergieunterschied zwischen dem elektronisch angeregten Triplett-Zustand T₁ und dem Grundzustand S₀ von mindestens 2.8 eV auszeichnen. Damit kommen diese cyclischen Phosphazenverbindungen der allgemeinen Formel (Ia) insbesondere als Matrixmaterialien, Blockermaterialien, Ladungstransportmaterialien und/oder Ladungsinjektionsmaterialien, bevorzugt Matrixmaterialien und/oder Blockermaterialien wie Lochblockermaterialien, für blaue Phosphoreszenz-Emitter in Frage. Somit sind die cyclischen Phosphazenverbindungen der Formel (I)einsetzbar als: Lochinjektions-, Lochleitungs-, Lochblocker-, Elektroneninjektions-, Elektronenleitungs-, Elektronenblocker-, Excitonenblocker- und/oder Matrixmaterialien. Bevorzugt werden die cyclischen Phosphazenverbindungen der Formel (I) als Matrixmaterialien und/oder Lochblockermaterialien eingesetzt.
Neben den vorstehend genannten elektronischen Eigenschaften ist es des Weiteren wesentlich, dass die als Materialien für OLEDs eingesetzten cyclischen Phosphazenverbindungen Glasübergangstemperaturen aufweisen, die einen Einsatz der cyclischen Phosphazenverbindungen in OLEDs ermöglichen. Die erfindungsgemäß eingesetzten cyclischen Phosphazenverbindungen weisen Glasübergangstemperaturen T_{g} von im Allgemeinen mindestens 50°C, bevorzugt mindestens 65°C.

Cyclische Phosphazenverbindungen der Formel (I), worin n 3 bedeutet (Cyclotriphosphazene), die mindestens einen substituierten stickstoffhaltigen Heteroarylrest, mindestens einen substituieren Arylrest, mindestens einen substituierten oder unsubstituierten Carbazolylrest oder mindestens einen substituierten oder unsubstituierten Azacarbazolylrest aufweisen, der mit mindestens einem der. P-Atome über ein Kohlenstoffatom verknüpft ist, sind im Stand der Technik nicht bekannt.

Ein weiterer Gegenstand der vorliegenden Erfindung ist die Verwendung cyclischer Phosphazenverbindungen der allgemeinen Formel (II) in OLEDS 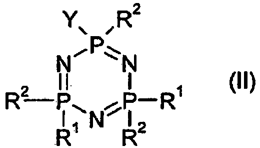 worin bedeuten
- Y: substituiertes C₆₋₁₄-Aryl, substituiertes stickstoffhaltiges Heteroaryl, das 5 bis 14 Ringatome aufweist, und über ein Kohlenstoffatom mit dem P-Atom des Phosphazenrings verknüpft ist, substituiertes oder unsubstituiertes Carbazolyl, das über ein Kohlenstoffatom mit dem P-Atom des Phosphazenrings verknüpft ist, oder substituiertes oder unsubstituiertes Azacarbazolyl, das über ein Kohlenstoffatom mit dem P-Atom des Phosphazenrings verknüpft ist;
- R¹, R²: unabhängig voneinander substituiertes oder unsubstituiertes Aryl, substituiertes oder unsubstituiertes Heteroaryl, substituiertes oder unsubstituiertes Alkyl, substituiertes oder unsubstituiertes Aryloxy, substituiertes oder unsubstituiertes Alkyloxy, F, wobei R¹ und R² jeweils gleich oder verschiesein können, bevorzugt unabhängig voneinander substituiertes oder unsubstituiertes Aryl, substituiertes oder unsubstituiertes Alkyl, substituiertes oder unsubstituiertes Aryloxy oder substituiertes oder unsubstituiertes Heteroaryl, besonders bevorzugt unabhängig voneinander substituiertes oder unsubstituiertes Aryl oder substituiertes oder unsubstituiertes Heteroaryl, wobei R¹ und R² jeweils gleich oder verschieden sein können; besonders bevorzugt unsubstituiertes oder substituiertes C₆₋₁₄-Aryl oder unsubstituiertes oder substituiertes Heteroaryl, das 5 bis 14 Ringatome aufweist, wobei R¹ und R² jeweils gleich oder verschieden sein können.

Bevorzugte Reste R¹ und R² entsprechen den bezüglich der cyclischen Phosphazenverbindungen der Formel (I) genannten bevorzugten Resten R¹ und R², wobei R¹ und R² nicht über eine Brücke verknüpft sind. Bevorzugte stickstoffhaltige Herteroarylreste und bevorzugte substituierte Arylreste entsprechen ebenfalls den bezüglich der cyclischen Phosphazenverbindungen der Formel (I) genannten Resten.

Wie in den cyclischen Phosphazenverbindungen der Formel (I) bzw. der Formeln (Ia) und (Iaa) ist es auch in den cyclischen Phosphazenverbindungen der Formel (II) bevorzugt, dass alle Reste R¹, R² und Y in den cyclischen Phosphazenverbindungen der Formel (II) dieselbe Bedeutung aufweisen. Ganz besonders bevorzugte cyclische Phosphazenverbindungen der Formel (II) sind cyclische Phosphazenverbindungen der Formel (IIa) 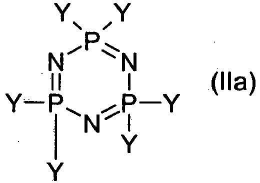 worin
- Y: substituiertes C₆₋₁₄-Aryl, substituiertes stickstoffhaltiges Heteroaryl, das 5 bis 14 Ringatome aufweist, und über ein Kohlenstoffatom mit dem P-Atom des Phosphazenrings verknüpft ist, substituiertes oder unsubstituiertes Carbazolyl, das über ein Kohlenstoffatom mit dem P-Atom des Phosphazenrings verknüpft ist, oder substituiertes oder unsubstituiertes Azacarbazolyl, das über ein Kohlenstoffatom mit dem P-Atom des Phosphazenrings verknüpft ist, bedeutet.

Bevorzugt bedeutet Y substituiertes C₆-Aryl oder substituiertes stickstoffhaltiges Heteroaryl mit 6 Ringatomen, substituiertes oder unsubstituiertes Carbazolyl oder substituiertes oder unsubstituiertes Azacarbazolyl. Geeignete Substituenten sind vorstehend genannt. Bevorzugte Substituenten sind C₁- bis C₆-Alkyl, z. B. Methyl, Ethyl, n-Propyl, iso-Propyl, n-Butyl, iso-Butyl, sek.-Butyl, tert.-Butyl, CN und C₁- bis C₆-Alkoxy, z. B. Methoxy, Ethoxy, Beispiele für geeignete Reste Y sind C₁-C₆-Alkylphenyl wie Methylphenyl, C₁-C₆-Dialkylphenyl wie Dimethylphenyl, C₁-C₆-Alkoxyphenyl wie Methoxyphenyl, C₁-C₆-Dialkoxyphenyl wie Dimethoxyphenyl, C₁-C₆-Alkylpyridyl wie Methylpyridyl, Carbazolyl, C₁-C₆-Alkylcarbazolyl wie Methylcarbazolyl, N-C₁-C₆-Alkylcarbazolyl wie N-Methylcarbazolyl, C₁-C₆-Dialkylcarbazolyl wie Dimethylcarbazolyl, Azacarbazolyl und N-C₁-C₆-Azacarbazolyl wie N-Methylazacarbazolyl.

Beispiele für besonders bevorzugte Verbindungen der Formel (IIa) sind die cyclischen Phosphazenverbindungen der Formeln (Iaab, Iaac und Iaaf bis Iaar).

Die cyclischen Phosphazenverbindungen der Formel (I) bzw. (II) werden bevorzugt durch Cyclokondensation von entsprechenden Phosphinamiden entsprechend dem in R. Appel et al. Chem. Ber. 106, 3455-3460 (1973) offenbarten Verfahren zur Herstellung von Cyclotriphosohazenen und Cyclotetraphosphazenen hergestellt. Die entsprechenden Phosphinamide können durch Amidierung der jeweiligen Phosphinsäuren erhalten werden. In dem folgenden Schema ist die Herstellung von cyclischen Phosphazenverbindungen der Formel (I) bzw. (II) beispielhaft an Cyclotriphosphazenen (n in den Verbindungen der Formel (I) = 3), worin die Reste R¹ und R² an allen P-Atomen jeweils dieselben Bedeutungen (R¹) aufweisen, beispielhaft dargestellt: 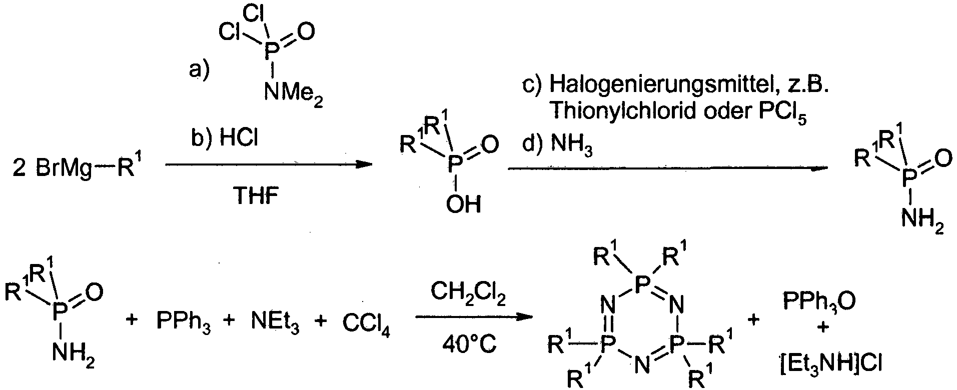 Geeignete Reaktionsbedingungen zur Herstellung der cyclischen Phosphazenverbindungen der Formel (I) bzw. (II) entsprechen den in V. Vincente et al. New J. Chem. 2004, 28, 418-424 genannten Reaktionsbedingungen zur Herstellung von Hexa-(2-thienyl)cyclotriphosphazenen bzw. Hexa-(3-thienyl)cyclotriphosphazenen und sind des Weiteren für die Herstellung ausgewählter besonders bevorzugter Verbindungen im Beispielteil der vorliegenden Anmeldung genannt. Des Weiteren sind geeignete Reaktionsbedingungen zur Herstellung von cyclischen Phosphazenen, worin R¹ und R² miteinander verknüpft sind (Spiro-Cyclophosphazenen), z. B. in C. Combes-Chamalet et al. J. Chem. Soc., Perkin Trans. 2, 1997, 15-18 offenbart.

Die erfindungsgemäß eingesetzten cyclischen Phosphazenverbindungen der Formel (I) bzw. die erfindungsgemäßen cyclischen Phosphazenverbindungen der Formel (II) können - wie vorstehend erwähnt - in verschiedenen Schichten der erfindungsgemäßen organischen Leuchtdiode eingesetzt werden, wobei geeignete Schichtfolgen in OLEDs dem Fachmann bekannt und nachstehend genannt sind.

In einer Ausführungsform betrifft die vorliegende Erfindung organische Leuchtdioden, worin die cyclischen Phosphazenverbindungen der Formel (I) bzw. (II) in der Licht-emittierenden Schicht als Matrix eingesetzt werden.

In einer weiteren Ausführungsform betrifft die vorliegende Erfindung eine erfindungsgemäße organische Leuchtdiode, worin die cyclischen Phosphazenverbindungen der Formel (I) bzw. (II) in der Blockschicht für Löcher als Loch/Excitonenblocker und/oder in der Elektronen-Injektionsschicht und/oder in der Elektronenleiterschicht eingesetzt werden. Es ist ebenfalls möglich, dass die Verbindungen der Formel (I) bzw. (II) in der Licht-emittierenden Schicht und/oder einer oder mehreren der vorstehend genannten Schichten vorliegen.

In einer weiteren Ausführungsform betrifft die vorliegende Erfindung eine erfindungsgemäße organische Leuchtdiode, worin die cyclischen Phosphazenverbindungen der Formel (I) bzw. (II) in der Blockschicht für Elektronen als Elektronen/Excitonenblocker und/oder in der Loch-Injektionsschicht und/oder in der Lochleiterschicht eingesetzt werden. Es ist ebenfalls möglich, dass die Verbindungen der Formel (I) bzw. (II) des Weiteren in der Licht-emittierenden Schicht und/oder einer oder mehreren der nachstehend genannten Schichten vorliegen.

In Abhängigkeit davon, in welcher Schicht die cyclischen Phosphazenverbindungen der Formel (I) bzw. (II) eingesetzt werden, weisen sie unterschiedliche bevorzugte Reste R¹ und R² und unterschiedliche Indizes n auf. Des Weiteren sind die Eigenschaften der cyclischen Phosphazenverbindungen (als Matrixmaterial, Loch-/Excitonenblocker-material oder Elektronen-/Excitonenblockermaterial, Loch- oder Elektronenleitermaterial und/oder Loch- oder Elektroneninjektionsmaterial) von den elektronischen Eigenschaften (relative Lagen der HOMOs und LUMOs) der jeweiligen in der erfindungsgemäßen OLED eingesetzten Schichten abhängig. Somit ist es möglich, durch geeignete Substitution der cyclischen Phosphazenverbindungen der Formel (I) bzw. (II) die HOMO und LUMO-Orbitallagen an die weiteren in der erfindungsgemäßen OLED eingesetzten Schichten anzupassen und so eine hohe Stabilität der OLED und damit eine lange operative Lebensdauer und gute Effizienzen zu erreichen.

Die Grundsätze betreffend die relativen Lagen von HOMO und LUMO in den einzelnen Schichten einer OLED sind dem Fachmann bekannt. Im Folgenden sind die Grundsätze beispielhaft bezüglich der Eigenschaften der Blockschicht für Elektronen und der Blockschicht für Löcher im Verhältnis zur Licht-emittierenden Schicht aufgeführt:

Das LUMO der Blockschicht für Elektronen liegt energetisch höher als das LUMO der in der Licht-emittierenden Schicht eingesetzten Materialien (sowohl des Emittermaterials auch gegebenenfalls eingesetzter Matrixmaterialien). Je größer die energetische Differenz der LUMOs der Blockschicht für Elektronen und der Materialien in der Licht-emittierenden Schicht ist, desto besser sind die Elektronen- und/oder Excitonenblockierenden Eigenschaften der Blockschicht für Elektronen. Geeignete Substitutionsmuster der als Elektronen- und/oder Excitonenblockermaterialien geeigneten cyclischen Phosphazenverbindungen der Formel (I) bzw. (II) sind somit unter anderem abhängig von den elektronischen Eigenschaften (insbesondere der Lage des LUMOs) der in der Licht-emittierenden Schicht eingesetzten Materialien.

Das HOMO der Blockschicht für Löcher liegt energetisch tiefer als die HOMOs der in der Licht-emittierenden Schicht vorliegenden Materialien (sowohl der Emittermaterialien als auch der gegebenenfalls vorliegenden Matrixmaterialien). Je größer die energetische Differenz der HOMOs der Blockschicht für Löcher und der in der Licht-emittierenden Schicht vorliegenden Materialien ist, desto besser sind die Loch- und/oder Excitonen-blockierenden Eigenschaften der Blockschicht für Löcher. Geeignete Substitutionsmuster der als Loch- und/oder Excitonenblockermaterialien geeigneten cyclischen Phosphazenverbindungen der Formel (I) bzw. (II) sind somit unter anderem abhängig von den elektronischen Eigenschaften (insbesondere der Lage der HOMOs) der in der Licht-emittierenden Schicht vorliegenden Materialien.

Vergleichbare Überlegungen betreffend die relative Lage der HOMOs und LUMOs der unterschiedlichen in der erfindungsgemäßen OLED eingesetzten Schichten gelten für die weiteren gegebenenfalls in der OLED eingesetzten Schichten und sind dem Fachmann bekannt.

Organische Leuchtdioden (OLEDs) sind grundsätzlich aus mehreren Schichten aufgebaut:
1. Anode (1)
2. Löcher-transportierende Schicht (2)
3. Licht-emittierende Schicht (3)
4. Elektronen-transportierende Schicht (4)
5. Kathode (5)

Es ist jedoch auch möglich, dass die OLED nicht alle der genannten Schichten aufweist, zum Beispiel ist eine OLED mit den Schichten (1) (Anode), (3) (Licht-emittierende Schicht) und (5) (Kathode) ebenfalls geeignet, wobei die Funktionen der Schichten (2) (Löcher-transportierende Schicht) und (4) (Elektronen-transportierende Schicht) durch die angrenzenden Schichten übernommen werden. OLEDs, die die Schichten (1), (2), (3) und (5) bzw. die Schichten (1), (3), (4) und (5) aufweisen, sind ebenfalls geeignet.

Die erfindungsgemäßen cyclischen Phosphazenverbindungen der Formel (I) können in einer oder mehreren verschiedenen Schichten einer OLED eingesetzt werden. Ein weiterer Gegenstand der vorliegenden Erfindung ist daher die Verwendung von cyclischen Phosphazenverbindungen der Formel (I) bzw. (II) in OLEDs.

Die cyclischen Phosphazenverbindungen der Formel (I) bzw. (II) werden in einer bevorzugten Ausführungsform in der Licht-emittierenden Schicht als Matrixmaterial eingesetzt. Ein weiterer Gegenstand der vorliegenden Erfindung ist daher eine organische Leuchtdiode enthaltend eine Licht-emittierende Schicht aufgebaut aus mindestens einem Matrixmaterial und mindestens einem Emittermaterial worin das mindestens eine Matrixmaterial mindestens eine cyclische Phosphazenverbindung der Formel (I) bzw. (II) enthält. Ein weiterer Gegenstand der vorliegenden Erfindung ist eine Lichtemittierende Schicht aufgebaut aus mindestens einem Matrixmaterial und mindestens einem Emittermaterial worin das mindestens eine Matrixmaterial mindestens eine cyclische Phosohazenverbindung der Formel (I) bzw. (II) enthält. Bevorzugte cyclische Phosphazenverbindungen der Formel (I) bzw. (II) sind vorstehend genannt.

Im Allgemeinen beträgt der Anteil der mindestens einen cyclischen Phosphazenverbindung der Formel (I) bzw. (II) in der Licht-emittierenden Schicht des erfindungsgemäßen OLEDs 10 bis 99 Gew.-%, bevorzugt 50 bis 99 Gew.-%, besonders bevorzugt 70 bis 97 Gew.-%. Der Anteil des mindestens einen Emitter-Materials in der Licht-emittierenden Schicht beträgt im Allgemeinen 1 bis 90 Gew.-%, bevorzugt 1 bis 50 Gew.-%, besonders bevorzugt 3 bis 30 Gew.-%, wobei die Anteile an der mindestens einen cyclischen Phosphazenverbindung der Formel (I) bzw. (II) und dem mindestens einen Emittermaterial 100 Gew.-% ergeben. Es ist jedoch auch möglich, dass die Licht-emittierende Schicht neben der mindestens einen cyclischen Phosphazenverbindung der allgemeinen Formel (I) bzw. (II) und dem mindestens einen Emittermaterial weitere Substanzen enthält, z. B. weiteres von den cyclischen Phosphazenverbindung der Formel (I) bzw. (II) verschiedenes Matrixmaterial, wobei dem Fachmann geeignete weitere Matrixmaterialien bekannt sind.

Die einzelnen der vorstehend genannten Schichten der OLED können wiederum aus 2 oder mehreren Schichten aufgebaut sein. Beispielsweise kann die Löchertransportierende Schicht aus einer Schicht aufgebaut sein, in die aus der Elektrode Löcher injiziert werden und einer Schicht, die die Löcher von der Lochinjektionsschicht weg in die Licht-emittierende Schicht transportiert. Die Elektronen-transportierende Schicht kann ebenfalls aus mehreren Schichten bestehen, zum Beispiel einer Schicht, worin Elektronen durch die Elektrode injiziert werden, und einer Schicht, die aus der Elektroneninjektionsschicht Elektronen erhält und in die Licht-emittierende Schicht transportiert. Diese genannten Schichten werden jeweils nach Faktoren wie Energieniveau, Temperaturresistenz und Ladungsträgerbeweglichkeit, sowie Energiedifferenz der genannten Schichten mit den organischen Schichten oder den Metallelektroden ausgewählt. Der Fachmann ist in der Lage, den Aufbau der OLEDs so zu wählen, dass er optimal an die erfindungsgemäßen, bevorzugt als Emittersubstanzen verwendeten Metallkomplexe angepasst ist.

Um besonders effiziente OLEDs zu erhalten, sollte das HOMO (höchstes besetztes Molekülorbital) der Loch-transportierenden Schicht mit der Arbeitsfunktion der Anode angeglichen sein und das LUMO (niedrigstes unbesetztes Molekülorbital) der elektronentransportierenden Schicht sollte mit der Arbeitsfunktion der Kathode angeglichen sein.

Geeignete Materialien für die vorstehend genannten Schichten (Anode, Kathode, Lochund Elektroneninjektionsmaterialien, Loch- und Elektronentransportmaterialien und Loch- und Elektronenblockermaterialien, Matrixmaterialien, Fluoreszenz- und Phosphoreszenzemitter) sind dem Fachmann bekannt und z. B. in H. Meng, N. Herron, Organic Small Molecule Materials for Organic Light-Emitting Devices in Organic Light-Emitting Materials and Devices, Ed.: Z. Li, H. Meng, Taylor & Francis, 2007, Chapter 3, Seiten 295 bis 411 genannt.

Die Anode (1) ist eine Elektrode, die positive Ladungsträger bereitstellt. Sie kann zum Beispiel aus Materialien aufgebaut sein, die ein Metall, eine Mischung verschiedener Metalle, eine Metalllegierung, ein Metalloxid oder eine Mischung verschiedener Metalloxide enthält. Alternativ kann die Anode ein leitendes Polymer sein. Geeignete Metalle umfassen die Metalle der Gruppen 11, 4, 5 und 6 des Periodensystems der Elemente sowie die Übergangsmetalle der Gruppen 8 bis 10. Wenn die Anode lichtdurchlässig sein soll, werden im Allgemeinen gemischte Metalloxide der Gruppen 12, 13 und 14 des Periodensystems der Elemente eingesetzt, zum Beispiel Indium-Zinn-Oxid (ITO). Es ist ebenfalls möglich, dass die Anode (1) ein organisches Material, zum Beispiel Polyanilin enthält, wie beispielsweise in Nature, Vol. 357, Seiten 477 bis 479 (11. Juni 1992) beschrieben ist. Zumindest entweder die Anode oder die Kathode sollten mindestens teilweise transparent sein, um das gebildete Licht auskoppeln zu können.

Geeignete Lochtransportmaterialien für die Schicht (2) der erfindungsgemäßen OLEDs sind zum Beispiel in Kirk-Othmer Encyclopedia of Chemical Technologie, 4. Auflage, Vol. 18, Seiten 837 bis 860, 1996 offenbart. Sowohl Löcher transportierende Moleküle als auch Polymere können als Lochtransportmaterial eingesetzt werden. Üblicherweise eingesetzte Löcher transportierende Moleküle sind ausgewählt aus der Gruppe bestehend aus 4,4'-Bis[N-(1-naphthyl)-N-phenyl-amino]biphenyl (α-NPD), N, N'-Diphenyl-N, N'-Bis(3-methylphenyl)-[1,1'-biphenyl]-4,4'-diamin (TPD), 1,1-Bis[(di-4-tolylamino)-phenyl]cyclohexan (TAPC), N, N'-Bis(4-methylphenyl)-N, N'-Bis(4-ethylphenyl)-[1,1'-(3,3`-dimethyl)biphenyl]-4,4`-diamin (ETPD), Tetrakis-(3-methylphenyl)-N,N,N',N'-2,5-phenylendiamin (PDA), α-Phenyl-4-N,N-diphenylaminostyrol (TPS), p-(Diethylamino)-benzaldehyddiphenylhydrazon (DEH), Triphenylamin (TPA), Bis[4-(N,N-diethylamino)-2-methylphenyl)(4-methyl-phenyl)methan (MPMP), 1-Phenyl-3-[p-(diethylamino)styryl]-5-[p-(diethylamino)phenyl]pyrazolin (PPR oder DEASP), 1,2-trans-Bis(9H-carbazol-9-yl)cyclobutan (DCZB), N,N,N',N'-tetrakis(4-methylphenyl)-(1,1'-biphenyl)-4,4'-diamin (TTB) 4,4',4"-tris(N,N-Diphenylamino)triphenylamin (TDTA) und Porphyrinverbindun-gen sowie Phthalocyaninen wie Kupferphthalocyanine. Üblicherweise eingesetzte Löcher transportierende Polymere sind ausgewählt aus der Gruppe bestehend aus Polyvinylcarbazolen, (Phenylmethyl)polysilanen, PEDOT (Poly(3,4-ethylendioxythiophen), bevorzugt PEDOT dotiert mit PSS (Polystyrolsulfonat), und Polyanilinen. Es ist eben-falls möglich, Löcher transportierende Polymere durch Dotieren Löcher transportierender Moleküle in Polymere wie Polystyrol und Polycarbonat zu erhalten. Geeignete Löcher transportierende Moleküle sind die bereits vorstehend genannten Moleküle.

Weiterhin können - in einer Ausführungsform - Metallkomplexe, z. B. Carben-Komplexe als Lochtransportmaterialien eingesetzt werden, wobei die Bandlücke des mindestens einen Lochtransportmaterials im Allgemeinen größer ist als die Bandlücke des eingesetzten Emittermaterials. Dabei ist unter Bandlücke im Sinne der vorliegenden Anmeldung die Triplett-Energie zu verstehen. Geeignete Carben-Komplexe sind z. B. Carben-Komplexe, wie sie in WO 2005/019373 A2, WO 2006/056418 A2, WO 2005/113704, WO 2007/115790 und WO 2007/115981 und in den älteren nicht vorveröffentlichten Europäischen Anmeldungen EP 07 118 677.9 und EP 08 153 303.6 beschrieben sind.

Die Licht-emittierende Schicht (3) enthält mindestens ein Emittermaterial. In einer bevorzugten Ausführungsform ist die Licht-emittierende Schicht aus mindestens einem Matrixmaterial und mindestens einem Emittermaterial aufgebaut, wobei das Matrixmaterial bevorzugt mindestens eine cyclische Phosphazenverbindung der Formel (I) bzw. (II) ist. Bevorzugte cyclische Phosphazenverbindungen sind vorstehend genannt. Da sich die erfindungsgemäß eingesetzten cyclischen Phosphazenverbindungen der Formel (I) bzw. (II) in einer besonders bevorzugten Ausführungsform durch einen Energieunterschied zwischen dem elektronisch angeregten Triplettzustand T₁ und dem Grundzustand S₀ von mindestes 2.8 eV auszeichnen, werden die erfindungsgemäß eingesetzten cyclischen Phosphazenverbindungen der Formel (I) bzw. (II) bevorzugt gemeinsam mit mindestens einem Blauemitter als Emittermaterial eingesetzt. Besonders bevorzugt liegt das Emissionsmaximum des Emittermaterials der Licht-emittierenden Schicht Licht im Bereich von 400 bis 500 nm ganz besonders bevorzugt 430 bis 490 nm. Bei dem mindestens einen Emittermaterial kann es sich grundsätzlich um einen Fluoreszenz- oder Phosphoreszenzemitter handeln, wobei geeignete Emittermaterialien dem Fachmann bekannt sind. Bevorzugt handelt es sich bei dem mindestens einen Emittermaterial um einen Phosphoreszenzemitter, ganz besonders bevorzugt um einen blaues Licht emittierenden Phosphoreszenzemitter. Die bevorzugt eingesetzten Phosphoreszenzmitter-Verbindungen basieren auf Metallkomplexen, wobei insbesondere die Komplexe der Metalle Ru, Rh, Ir, Pd und Pt, vor allem die Komplexe des Ir Bedeutung erlangt haben. Die erfindungsgemäß verwendeten cyclischen Phosphazenverbindungen der Formel (I) bzw. (II) sind besonders für den Einsatz gemeinsam mit solchen Metallkomplexen geeignet.

Geeignete Metallkomplexe zur Verwendung in den erfindungsgemäßen OLEDs sind z. B. in den Schriften WO 02/60910 A1, US 2001/0015432 A1, US 2001/0019782 A1, US 2002/0055014 A1, US 2002/0024293 A1, US 2002/0048689 A1, EP 1 191 612 A2, EP 1 191 613 A2, EP 1 211 257 A2, US 2002/0094453 A1, WO 02/02714 A2, WO 00/70655 A2, WO 01/41512 A1, WO 02/15645 A1, WO 2005/019373 A2, WO 2005/113704 A2, WO 2006/115301 A1, WO 2006/067074 A1, WO 2006/056418, WO 2007/115981, WO 2007/115970, WO 2008/000726, WO 2008/000727, WO 2007/018067, US 2007/0108891, WO 2007/058080, WO 2007/058104, WO 2006/106842, WO 2007/058255, WO 2007/069542, JP 2007/084635, JP 2007/045742, JP 2006/120762, JP 2006/120905, JP 2007/051243, JP 2007/059688, JP 2007/123392, JP 2007/051243, US 2007/0196690, WO 2006/126389, WO 2007/023659, WO 2007/029461, WO 2007/052431, WO 2007/060826, WO 2007/069539, WO 2005/123873, WO 2006/046980, WO 2006/121811, WO 2007/095118, WO 2005/097941, WO 2005/097942, WO 2005/097943 und den nicht vorveröffentlichten europäischen Anmeldungen EP 07 118677.9 und EP 08 153303.6 sowie in der nicht vorveröffentlichten älteren PCT-Anmeldung mit dem Aktenzeichen PCT/EP2008/054087 beschrieben.

Weitere geeignete Metallkomplexe sind die kommerziell erhältlichen Metallkomplexe Tris(2-phenylpyridin)iridium(III), Iridium(III)tris(2-(4-tolyl)pyridinato-N,C²'), Iridium(III)tris(1-phenylisochinolin), Iridium(III)bis(2-2'-benzo-thienyl)pyridinatoN,C³')(acetylacetonat), Iridium(III)bis(2-(4,6-difluorophenyl)pyridinato-N,C²)picolinat, Iridium(III)bis(1-phenylisochinolin)(acet ylacetonat), Iridium(III)bis(dibenzo[f,h]chinoxalin)(acetylacetonat), Iridium(III)bis(2-methyldi-benzo[f,h]chinoxalin)-(acetylacetonat) und Tris(3-methyl-1-phenyl-4-trimethyl-acetyl-5-pyrazolin)terbium(III).

Des Weiteren sind die folgenden kommerziell erhältlichen Materialien geeignet: Tris(dibenzoylacetonato)-mono(phenanthrolin)-europium(III), Tris(dibenzoylmethan)-mono(phenanthrolin)europium(III), Tris(dibenzoylmethan)-mono(5-aminophenanthrolin)europium(III), Tris(di-2-naphthoy)methan)-mono(phenanthrolin)-europium(III), Tris(4-bromobenzoylmethan)-mono(phenanthrolin)-europium(III), Tris(di-biphenyl-methan)-mono(phenanthrolin)-europium(III), Tris(dibenzoylmethan)-mono(4,7-diphenyl-phenanthrolin)-europium(III), Tris(dibenzoylmethan)-mono(4,7-di-methylphenanthrolin)-europium(III), Tris(dibenzoylmethan)-mono(4,7-dimethyl-phenanthrolin-disulfonsäure)-europium(III)-dinatriumsalz, Tris[di(4-(2-(2-ethoxy-ethoxy)ethoxy)benzoylmethan)]-mono(phenanthrolin)-europium(III) und Tris[di[4-(2-(2-ethoxyethoxy)ethoxy)benzoylmethan)]mono-(5-aminophenanthrolin)-europium(III).

Besonders bevorzugt werden als Emittermaterialien Metallkomplexe, z. B. Metallkomplexe, die in den vorstehend genannten Dokumenten offenbart sind, eingesetzt, die ein Emissionsmaximum im Bereich von 400 bis 500 nm, bevorzugt 430 bis 490 nm, aufweisen.

Weitere bevorzugt geeignete Triplett-Emitter sind Carbenkomplexe. In einer bevorzugten Ausführungsform der vorliegenden Erfindung werden die cyclischen Phosphazenverbindungen der Formel (I) bzw. (II) in der Licht-emittierenden Schicht als Matrixmaterial gemeinsam mit Carbenkomplexen, ganz besonders bevorzugt gemeinsam mit blau emittierenden Carbenkomplexen als Triplett-Emitter eingesetzt. Geeignete Carbenkomplexe sind dem Fachmann bekannt und in einigen der vorstehend genannten Anmeldungen und nachstehend genannt.

In einer weiteren bevorzugten Ausführungsform werden die-cyclischen Phosphazenverbindungen der Formel (I) bzw. (II) als Loch/Excitonen-Blockermaterial gemeinsam mit Carbenkomplexen als Triplett-Emitter eingesetzt. Die cyclischen Phosphazenverbindungen der Formel (I) bzw. (II) können des Weiteren sowohl als Matrixmaterialien als auch als Loch-/Excitonenblockermaterialien gemeinsam mit Carbenkomplexen als Triplett-Emitter eingesetzt werden.

Geeignete Metallkomplexe zur Verwendung zusammen mit den cyclischen Phosphazenverbindungen der Formel (I) bzw. (II) als Matrixmaterialien und/oder Loch-/Excitonenblockermaterialien und/oder Ladungstransportmaterialien und/oder Ladungsinjektionsmaterialien, bevorzugt Matrixmaterialien und/oder Lochblockermaterialien, in OLEDs sind somit z. B. auch Carben-Komplexe, wie sie in WO 2005/019373 A2, WO 2006/056418 A2 und WO 2005/113704, WO 2007/115970 und in WO 2007/115981 sowie in den älteren, nicht vorveröffentlichten europäischen Anmeldungen EP 07 118677.9 und EP 08 153303.6 beschrieben sind. Auf die Offenbarung der genannten WO- und EP-Anmeldungen wird hierbei explizit Bezug genommen und diese Offenbarungen sollen in den Inhalt der vorliegenden Anmeldung als mit einbezogen gelten.

Geeignete Elektronentransportmaterialien für die Schicht (4) der erfindungsgemäßen OLEDs umfassen mit oxinoiden Verbindungen chelatisierte Metalle wie Tris(8-hydroxychinolato)aluminium (Alq₃), Verbindungen auf Phenanthrolinbasis wie 2,9-Dimethyl, 4,7-Diphenyl-1, 10-phenanthrolin (DDPA = BCP) oder 4,7-Diphenyl-1,10-phenanthrolin (DPA) und Azolverbindungen wie 2-(4-Biphenylyl)-5-(4-t-butylphenyl)-1,3,4-oxadiazol (PBD) und 3-(4-Biphenylyl)-4-phenyl-5-(4-t-butylphenyl)-1,2,4-triazol (TAZ). Dabei kann die Schicht (4) sowohl zur Erleichterung des Elektronentransports dienen als auch als Pufferschicht oder als Sperrschicht, um ein Quenchen des Excitons an den Grenzflächen der Schichten der OLEDs zu vermeiden. Vorzugsweise verbessert die Schicht (4) die Beweglichkeit der Elektronen und reduziert ein Quenchen des Excitons.

Von den vorstehend als Lochtransportmaterialien und Elektronen transportierende Materialien genannten Materialien können einige mehrere Funktionen erfüllen. Zum Beispiel sind einige der Elektronen leitenden Materialien gleichzeitig Löcher blockende Materialien, wenn sie ein tief liegendes HOMO aufweisen.

Die Ladungstransportschichten können auch elektronisch dotiert sein, um die Transporteigenschaften der eingesetzten Materialien zu verbessern, um einerseits die Schichtdicken großzügiger zu gestalten (Vermeidung von Pinholes/Kurzschlüssen) und um andererseits die Betriebsspannung des Devices zu minimieren. Beispielsweise können die Lochtransportmaterialien mit Elektronenakzeptoren dotiert werden, zum Beispiel können Phthalocyanine bzw. Arylamine wie TPD oder TDTA mit Tetrafluorotetracyano-chinodimethan (F4-TCNQ) dotiert werden. Die Elektronentransportmaterialien können zum Beispiel mit Alkalimetallen dotiert werden, beispielsweise Alq₃ mit Lithium. Die elektronische Dotierung ist dem Fachmann bekannt und zum Beispiel in W. Gao, A. Kahn, J. Appl. Phys., Vol. 94, No. 1, 1 July 2003 (p-dotierte organische Schichten); A. G. Werner, F. Li, K. Harada, M. Pfeiffer, T. Fritz, K. Leo, Appl. Phys. Lett., Vol. 82, No. 25, 23 June 2003 und Pfeiffer et al., Organic Electronics 2003, 4, 89 - 103 offenbart.

Die Kathode (5) ist eine Elektrode, die zur Einführung von Elektronen oder negativen Ladungsträgern dient. Die Kathode kann jedes Metall oder Nichtmetall sein, das eine geringere Arbeitsfunktion aufweist als die Anode. Geeignete Materialien für die Kathode sind ausgewählt aus der Gruppe bestehend aus Alkalimetallen der Gruppe 1, zum Beispiel Li, Cs, Erdalkalimetallen der Gruppe 2, Metallen der Gruppe 12 des Periodensystems der Elemente, umfassend die Seltenerdmetalle und die Lanthanide und Aktinide. Des Weiteren können Metalle wie Aluminium, Indium, Calcium, Barium, Samarium und Magnesium sowie Kombinationen davon eingesetzt werden. Weiterhin können Lithium enthaltende organometallische Verbindungen oder LiF zwischen der organischen Schicht und der Kathode aufgebracht werden, um die Betriebsspannung (Operating Voltage) zu vermindern.

Die OLED gemäß der vorliegenden Erfindung kann zusätzlich weitere Schichten enthalten, die dem Fachmann bekannt sind. Beispielsweise kann zwischen der Schicht (2) und der Licht emittierenden Schicht (3) eine Schicht aufgebracht sein, die den Transport der positiven Ladung erleichtert und/oder die Bänderlücke der Schichten aneinander anpasst. Alternativ kann diese weitere Schicht als Schutzschicht dienen. In analoger Weise können zusätzliche Schichten zwischen der Licht emittierenden Schicht (3) und der Schicht (4) vorhanden sein, um den Transport der negativen Ladung zu erleichtern und/oder die Bänderlücke zwischen den Schichten aneinander anzupassen. Alternativ kann diese Schicht als Schutzschicht dienen.

In einer bevorzugten Ausführungsform enthält die erfindungsgemäße OLED zusätzlich zu den Schichten (1) bis (5) mindestens eine der im Folgenden genannten weiteren Schichten:
- eine Loch-Injektionsschicht zwischen der Anode (1) und der Löchertransportierenden Schicht (2);
- eine Blockschicht für Elektronen und/oder Excitonen zwischen der Löchertransportierenden Schicht (2) und der Licht-emittierenden Schicht (3);
- eine Blockschicht für Löcher und/oder Excitonen zwischen der Licht-emittierenden Schicht (3) und der Elektronen-transportierenden Schicht (4);
- eine Elektronen-Injektionsschicht zwischen der Elektronen-transportierenden Schicht (4) und der Kathode (5).

Wie bereits vorstehend erwähnt, ist es jedoch auch möglich, dass die OLED nicht alle der genannten Schichten (1) bis (5) aufweist, zum Beispiel ist eine OLED mit den Schichten (1) (Anode), (3) (Licht-emittierende Schicht) und (5) (Kathode) ebenfalls geeignet, wobei die Funktionen der Schichten (2) (Löcher-transportierende Schicht) und (4) (Elektronen-transportierende Schicht) durch die angrenzenden Schichten übernommen werden. OLEDs, die die Schichten (1), (2), (3) und (5) bzw. die Schichten (1), (3), (4) und (5) aufweisen, sind ebenfalls geeignet.

Dem Fachmann ist bekannt, wie er (zum Beispiel auf Basis von elektrochemischen Untersuchungen) geeignete Materialien auswählen muss. Geeignete Materialien für die einzelnen Schichten sowie geeignete OLED-Aufbauten sind dem Fachmann bekannt und z. B. in WO2005/113704 offenbart.

Des Weiteren kann jede der genannten Schichten der erfindungsgemäßen OLED aus zwei oder mehreren Schichten aufgebaut sein. Des Weiteren ist es möglich, dass einige oder alle der Schichten (1), (2), (3), (4) und (5) oberflächenbehandelt sind, um die Effizienz des Ladungsträgertransports zu erhöhen. Die Auswahl der Materialien für jede der genannten Schichten ist bevorzugt dadurch bestimmt, eine OLED mit einer hohen Effizienz zu erhalten.

Die Herstellung der erfindungsgemäßen OLED kann nach dem Fachmann bekannten Methoden erfolgen. Im Allgemeinen wird die OLED durch aufeinander folgende Dampfabscheidung (Vapor deposition) der einzelnen Schichten auf ein geeignetes Substrat hergestellt. Geeignete Substrate sind zum Beispiel Glas oder Polymerfilme. Zur Dampfabscheidung können übliche Techniken eingesetzt werden wie thermische Verdampfung, Chemical Vapor Deposition und andere. In einem alternativen Verfahren können die organischen Schichten aus Lösungen oder Dispersionen in geeigneten Lösungsmitteln beschichtet werden, wobei dem Fachmann bekannte Beschichtungstechniken angewendet werden. Zusammensetzungen, die neben dem mindestens einen erfindungsgemäßen Metallkomplex ein polymeres Material in einer der Schichten der OLED, bevorzugt in der Licht-emittierenden Schicht, aufweisen, werden im Allgemeinen mittels lösungsverarbeitenden Verfahren als Schicht aufgebracht.

Im Allgemeinen haben die verschiedenen Schichten folgende Dicken: Anode (1) 500 bis 5000 Å, bevorzugt 1000 bis 2000 Å; Löcher-transportierende Schicht (2) 50 bis 1000 Å, bevorzugt 200 bis 800 Å, Licht-emittierende Schicht (3) 10 bis 1000 Å, bevorzugt 100 bis 800 Å, Elektronen transportierende Schicht (4) 50 bis 1000 Å, bevorzugt 200 bis 800 Å, Kathode (5) 200 bis 10.000 Å, bevorzugt 300 bis 5000 Å. Die Lage der Rekombinationszone von Löchern und Elektronen in der erfindungsgemäßen OLED, und somit das Emissionsspektrum der OLED können durch die relative Dicke jeder Schicht beeinflusst werden. Das bedeutet, die Dicke der Elektronentransportschicht sollte bevorzugt so gewählt werden, dass die Elektronen/Löcher Rekombinationszone in der Licht-emittierenden Schicht liegt. Das Verhältnis der Schichtdicken der einzelne Schichten in der OLED ist von den eingesetzten Materialien abhängig. Die Schichtdicken von gegebenenfalls eingesetzten zusätzlichen Schichten sind dem Fachmann bekannt.

Durch Einsatz der cyclischen Phosphazenverbindungen der Formel (I) bzw. (II) in mindestens einer Schicht der erfindungsgemäßen OLED, z.B. als Matrixmaterial in der Licht-emittierenden Schicht der erfindungsgemäßen OLEDs und/oder als Loch-/Excitonenblockermaterial in der Blockschicht für Löcher und/oder Excitonen oder als Ladungstransportmaterial und/oder als Ladungsinjektionsmaterial und/oder als Elektronenblockermaterial, können OLEDs mit guter Effizienz und Lebensdauer erhalten werden. Die Effizienz der erfindungsgemäßen OLEDs kann des Weiteren durch Optimierung der anderen Schichten verbessert werden. Beispielsweise können hoch effiziente Kathoden wie Ca, Ba oder LiF eingesetzt werden. Geformte Substrate und neue Löcher-transportierende Materialien, die eine Reduktion der Operationsspannung oder eine Erhöhung der Quanteneffizienz bewirken, sind ebenfalls in den erfindungsgemäßen OLEDs einsetzbar. Des Weiteren können zusätzliche Schichten in den OLEDs vorhanden sein, um die Energieniveaus der verschiedenen Schichten einzustellen und um Elektrolumineszenz zu erleichtern.

Die erfindungsgemäßen OLEDs können in allen Vorrichtungen eingesetzt werden, worin Elektrolumineszenz nützlich ist. Geeignete Vorrichtungen sind bevorzugt ausgewählt aus stationären und mobilen Bildschirmen. Stationäre Bildschirme sind z. B. Bildschirme von Computern, Fernsehern, Bildschirme in Druckern, Küchengeräten sowie Reklametafeln, Beleuchtungen und Hinweistafeln. Mobile Bildschirme sind z. B. Bildschirme in Handys, Laptops, Kameras, insbesondere Digitalkameras, Fahrzeugen sowie Zielanzeigen an Bussen und Bahnen.

Weiterhin können die cyclischen Phosphazenverbindungen der Formel (I) bzw. (II) in OLEDs mit inverser Struktur eingesetzt werden. Der Aufbau von inversen OLEDs und die üblicherweise darin eingesetzten Materialien sind dem Fachmann bekannt.

Die nachfolgenden Beispiele erläutern die Erfindung zusätzlich:

### Beispiele

### I. Herstellung von cyclischen Phosphazenverbindungen der allgemeinen Formel 1

### I.1 Geräte und verwendete Chemikalien

### I.1.1 Geräte

| | |
|---|---|
| ¹H-, ¹³C-, ³¹P-NMR-Spektroskopie | BRUKER AC 250 (250 MHz) |
| Massenspektrometrie | Finnigan Mat 8500, MATT 112 S Varian mit EI-Ionisation |
| Oligomer-GPC | Waters Modell 510 mit |
| | UV-Detektor (WATERS 486, 254 nm) |
| | RI-Detektor (WATERS 410) |
| | Säulen: PL-Gel (Porengröße 100, 500 Å; Partikelgröße: 5µm, Länge: 60 cm) |
| | Laufmittel: THF, Kalibrierung mit Polystyrolstandards Fließgeschwindigkeit: 0.5 mL/min |
| Fluoreszenzspektroskopie | SHIMADZU RF-5301 PC Spectrofluorometer |
| UV/VIS-Spektroskopie | HITACHI U-3000 Spectrophotometer |
| Spin-Coater | B 0574, HAMATECH GmbH |
| | HS 485 Processcontrol, HAMATECH GmbH |
| Thermogravimetrie | NETSCH Simultane Thermoanalyse, |
| | STR 409 C; Heizrate: 10 K/min |
| DSC | Diamond DSC |
| Flash-Chromatographie | Kieselgel, 0.04 - 0.063 mm, Merck |
| Dünnschichtchromatographie | Dünnschichtkarten Polygram SIL G/UV₂₅₄, Roth |
| Profilometer | Sloan Dektak 3030ST. Rev. 5.2/5.1/F5.0 |
| Aufdampfanlage | PLS 500, Balzers |
| Elektrolumineszenz Detektor | MINOLTA Luminance Meter LS-100 |
| OLED-Charakterisierung | Grundig Electronics PN300, KEITHLEY Model 2000 Multimeter |

### l.1.2 Verwendete Lösungsmittel und Chemikalien

Alle kommerziellen Chemikalien werden nach Erhalt ohne weitere Reinigung eingesetzt. Alle Lösungsmittel für Reaktionen und Reinigung werden vor Gebrauch einmal destilliert, Tetrahydrofuran (THF) wurde zusätzlich über Kalium destilliert.

### I.2 Synthesevorschriften und Charakterisierung der Verbindungen

### I.2.1 Bis(3,5-dimethylphenyl)phosphinsäure

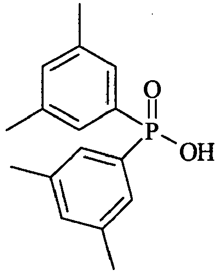

1-Brom-3,5-dimethylbenzol (13.63 g, 71.44 mmol, 97%) wird zu Magnesiumspänen (1.74 g, 71.44 mmol) in wasserfreiem THF (80 mL) bei Raumtemperatur zugetropft. Nachdem sich das Magnesium komplett aufgelöst hat, wird eine Lösung aus N,N-Dimethylphosphoryl dichlorid N(Me)₂P(O)Cl₂ (4.33 mL, 35.72 mmol) in wasserfreiem THF (10 mL) zugetropft. Zum Abtrennen des bei der Reaktion entstandenen Salzes wird das Reaktionsgemisch nach zwei Stunden in eine eisgekühlte Ammoniumchloridlösung (30 g in 500 mL Wasser) gegeben. In einer Destillationsapparatur wird anschließend das THF entfernt und der Rückstand mit konzentrierter HCl (75 mL) bei 80°C gekocht. Der resultierende weiße Feststoff wird in wässriger NaOH (5.4 g, 300 mL Wasser) aufgelöst und die wässrige Phase zweimal mit Diethylether extrahiert. Die klare wässrige Phase wird durch Zugabe von konzentrierter HCl erneut angesäuert, um die Phosphinsäure als weißen Feststoff in 70 % Ausbeute (6.81 g) auszufällen.

| Charakterisierung: | |
|---|---|
| ¹H-NMR (CDCl₃) | δ(ppm): 11.35 (s, 1 H, OH), 7.35 (d, 4H, *o*-Hs), 7.11 (s, 2H, *p-*Hs), 2.28 (m, 12H, CH₃). |
| MS | m/z (%): 274 (M⁺, 100), 259 (M-CH₃⁺, 39), 136 (M²⁺, 5). |

### I.2.2 Bis(4-methoxyphenyl)phosphinsäure

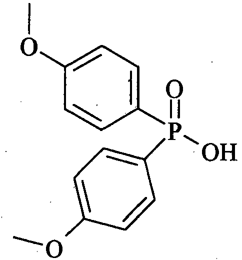

Die Synthese erfolgt analog zur oben beschriebenen Synthese von Bis(3,5-dimethyl-phenyl)phosphinsäure.

| | |
|---|---|
| 9.03 mL (71.44 mmol) | 4-Bromanisol, 99% |
| 1,74 g (71,44 mmol) | Magnesium |
| 4.33 mL (35,72 mmol) | N,N-Dimethylphosphoryldichlorid 98% |
| 80 mL | THF (abs.) |

Ausbeute: 54 % (5.36 g) weißer Feststoff.

| Charakterisierung: | |
|---|---|
| ¹H-NMR (CDCl₃) | δ (ppm): 7.77 (br s, 1H, OH), 7.62 (dd, 4H, *meta*-Hs), 6.83 (dd, 4H, *ortho*-Hs), 3.79 (m, 6H, methoxy). |
| MS | m/z (%): 278 (M⁺, 100), 263 (M-CH₃⁺, 19), 247 (M- OCH₃, 8), 107 (M-(C₆H₄-OCH₃, 15). |

### I.2.3 Bis(3,5-dimethylphenyl)phosphinamid

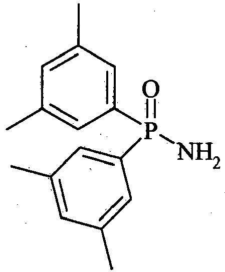

Destilliertes Thionylchlorid (0.87 mL, 12 mmol) wird zu einer Suspension aus Bis(3,5-dimethylphenyl)phosphinsäure (1 g, 3.65 mmol) in trockenem Toluol (25 mL) bei 55-60°C zugetropft und die entstandene klare Lösung 20 min gerührt. Nach Abdestillieren des überschüssigen Thionlychlorids wird Ammoniakgas in die Lösung bei -20°C eingeleitet. Um das entstandene Salz (NH₄Cl) abzutrennen, wird die organische Phase mit Wasser gewaschen. Vor der Extraktion mit THF wird die Dichte der wässrigen Phase durch Zugabe von Natriumchlorid erhöht. Nach Trocknung der organischen Phase mit Na₂SO₄ wird das Lösungsmittel am Rotationsverdampfer entfernt. Die Ausbeute liegt bei 0.98 g (98 %).

| Charakterisierung: | |
|---|---|
| ¹H-NMR (CDCl₃) | δ (ppm): 7.53 (d, 4H, o-Hs), 7.11 (s, 2H, *p*-Hs), 3.01 (br s, 2H, NH₂), 2.32 (m, 12H, CH₃). |
| MS | m/z (%): 273 (M⁺, 100), 257 (M-NH₂⁺, 19). |

### I.2.4 Bis(3,5-dimethylphenyl)phosphinamid

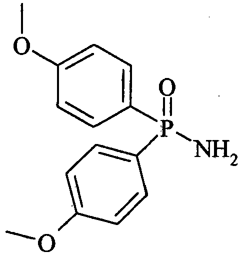

Die Synthese erfolgt analog zur oben beschriebenen Synthese von Bis(3,5-dimethyl-phenyl)phosphinamid.

| | |
|---|---|
| 1.0g (3.59 mmol) | Bis(4-methoxyphenyl)phosphinsäure |
| 0.78 mL (10.77 mmol) | Thionylchlorid |
| 25 mL | trockenes Toluol |

Ausbeute: 90 % (0.89 g) gelblicher Feststoff.

| Charakterisierung: | |
|---|---|
| ¹H-NMR (CDCl₃) | δ(ppm): 7.83 (dd or m, 4H, *meta-*Hs), 6.92 (dd or m, 4H, *orto-*Hs), 3.81 (m, 3H, methoxy), 3.08 (br s, 1.7Hs, NH₂). |
| MS | m/z (%):277(M⁺, 100), 170 (M-(methoxyphenyl)⁺, 45). |

### I.2.5 Hexaphenylcyclotriphosphazen (1a)

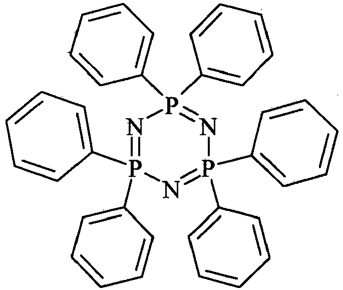

Eine Suspension aus Diphenylphosphinamid (2.0 g, 9.22 mmol), Triphenylphosphin (2.9 g, 11.1 mmol), Tetrachlorkohlenstoff (0.89 mL, 9.22 mmol) und Triethylamin (1.3 mL, 9.22 mmol) in trockenem Dichlormethan (25 mL) wird für fünf Stunden unter Rückfluss gekocht. Das Lösungsmittel wird am Rotationsverdampfer entfernt. Nach Säulenchromatographie mit Hexan : THF (1 : 1) als Laufmittel werden 1.2 g (65 %) Hexaphenylcyclotriphosphazen als weiße Substanz erhalten. Weitere Aufreinigung erfolgt mittels Sublimation.

| Charakterisierung: | |
|---|---|
| ¹H-NMR (CDCl₃) | δ (ppm): 7.76 (dd, 12H, *ortho*-Hs), 7.30 (m, 18H, *meta-*/*para-*Hs). |
| ¹³C-NMR (CDCl₃) | δ (ppm): 138.55 (dt, C*_{P-bound}*), 130.86 (ps d, C*ₘₑₜₐ*), 130.44 (m, C*ₚₐᵣₐ*), 127.98 (ps d, *C_{Ortho}*). |
| ³¹P-NMR (CDCl₃) | δ(ppm): 16.24 |
| Elementaranalyse | Berechnet: C: 72.36 %, H: 5.06 %, N: 7.03 %, P: 15.55 % |
| | Gefunden: C: 72.38 %, H: 5.08%, N: 7.07 %, P: 15.49 % |
| MS | m/z (%): 597 (M⁺, 100), 520 (M-Ph⁺, 74), 299 (M²⁺, 12), 260 (M²⁺-Ph, 18), 77 (Ph⁺, 5). |

### I.2.6 Hexa-(3,5-dimethylphenyl)-cyclotriphosphazen (1b)

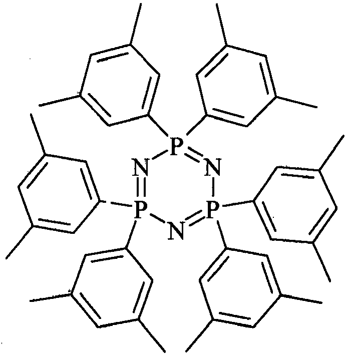

Die Synthese erfolgt analog zur oben beschriebenen Synthese von Hexaphenylcyclotriphosphazen. Die Reaktionszeit beträgt 22 Stunden.

| | |
|---|---|
| 1.0 g (3.66 mmol) | Bis(3,5-dimethylphenyl)phosphinamid |
| 1.15 g (4.39 mmol) | Triphenylphosphin |
| 0.53 mL (5.49 mmol) | Tetrachlorkohlenstoff |
| 0.76 mL (5.49 mmol) | Triethylamin |
| 25 mL | trockenes Dichlormethane |

Die Säulenchromatographie mit Hexan : THF (10 : 1) als Laufmittel liefert 660 mg (71 %) an Hexa-(3,5-dimethylphenyl)-cyclotriphosphazen als weißen Feststoff. Weitere Aufreinigung erfolgt mittels Sublimation.

| Charakterisierung: | |
|---|---|
| ¹H-NMR (CDCl₃) | δ (ppm): 7.40 (d, 12H, *ortho*-Hs), 6.98(s, 6H, *para*-Hs), 2.21 (m, 36H, CH₃). |
| ¹³C-NMR (CDCl₃) | δ (ppm): 138.35 (dt, C*_{P-bound}*), 137.16 (ps d, C*ₘₑₜₐ*), 131.97 (d, C*ₚₐᵣₐ*), 128.90 (d, C*ₒᵣₜₕₒ*)21.34 (s ,methyl). |
| Elementaranalyse | Berechnet: C: 75.27 %, H: 7.11 %, N: 5.49 %, P: 12.13 % |
| | Gefunden: C: 75.14 %, H: 7.04 %, N: 5.54 %, P: 12.18% |
| MS | m/z (%): 765 (M⁺, 100), 660 (M-Ph⁺, 45), 382 (M²⁺, 10), 105 (meta-Xylol⁺, 10). |

### I.2.7 Hexa-(4-methoxyphenyl)-cyclotriphosphazen (1c)

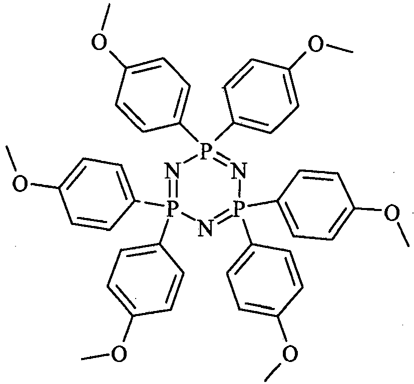

Die Synthese erfolgt analog zur oben beschriebenen Synthese von Hexaphenylcyclotriphosphazen. Die Reaktionszeit beträgt 14 Stunden.

| | |
|---|---|
| 1.11 g (4 mmol) | Bis(4-methhtoxyphenyl)-phosphinamid |
| 1.26 g (4.8 mmol) | Triphenylphosphin |
| 0.58 mL (6 mmol) | Tetrachlorkohlenstoff |
| 0.83 mL (6 mmol) | Triethylamin |
| 35 mL | trockenes Dichlormethan |

Die Säulenchromatographie mit Hexan : Ethylacetat (1: 1) als Laufmittel liefert 516 mg (50 %) an Hexa-(4-methoxyphenyl)-cyclotriphosphazen als weißen Feststoff.

| Charakterisierung: | |
|---|---|
| ¹H-NMR (CDCl₃) | δ (ppm): 7.64 (dd or m, 12H, *meta-*Hs), 6.80 (dd or m, 12H, *ortho-*Hs), 3.78 (m, 18H, methoxy). |
| ¹³C-NMR (CDCl₃) | δ (ppm): 161.24 (m, C*ₚₐᵣₐ*), 132.71 (ps d, C*ₘₑₜₐ*), 130.84 (dt, C*_{Pbound}*), 113.39 (ps d, C*ₒᵣₜₕₒ*) , 55.31 (s, methoxy). |
| Elementaranalyse | Berechnet: C: 64.86 %, H: 5.44 %, N: 5.40 %, O: 12.34 %, P: 11.95% |
| | Gefunden: C: 65.28 %, H: 5.44 %, N: 5.76 %, O:12.69 %, P: 10.81% |
| MS | m/z (%): 777 (M⁺, 99), 670 (M-(methoxyphenyl))⁺, 45), 388 (M²⁺ 22). |

### II OLED enthaltend Hexaphenylcyclotriphosphazen (1a) als Matrixmaterial

### II.1 Diodenaufbau

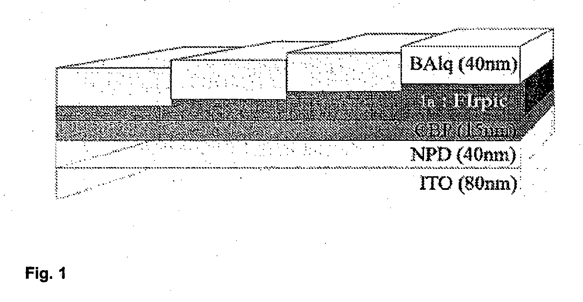

Als Diodenaufbau wird die in Figur 1 dargestellte Schichtabfolge verwendet.
In Figur 1 bedeuten:

| | |
|---|---|
| BAlq (40 nm) | eine 40 nm dicke lochblockierende Elektronenleiterschicht aus Aluminum(III)-bis-(2-methyl-8-chinolinat)-4-phenylphenolat (A) |
| 1a : Flrpic | Emissionsschicht aus 15 Gew.-% Flrpic (B) in 1a als Matrix (Schichtdicken von 20 und 26 nm wie nachstehend erwähnt) |
| CBP (15 nm) | eine 15 nm dicke Schicht aus 4,4'-bis(carbazol-9-yl)biphenyl (C) |
| NPD (40 nm) | eine 40 nm dicke Lochtransportschicht aus N,N'-Diphenyl-N,N'-bis(1-naphthyl)-1,1'-biphenyl-4,4"-diamin (D) |
| ITO (80 nm) | eine 80 nm dicke Schicht aus Indium-Zinn-Oxid |

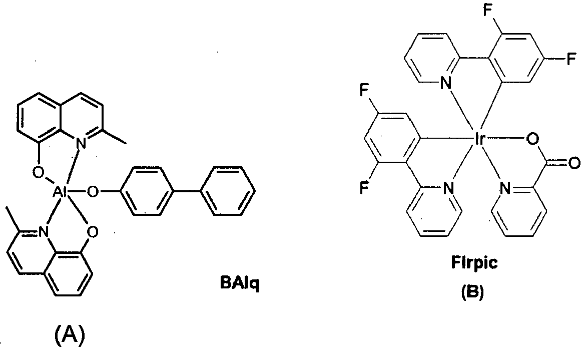

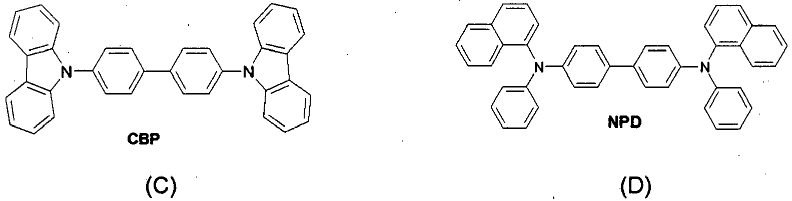

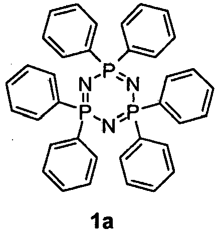

### II.2 Herstellung der OLED

Auf ein ITO-Substrat werden 40 nm NPD als Lochtransportmaterial aufgedampft. Die darauf folgende 15 nm dicke CBP-Schicht fungiert hierbei als energetische Stufe zwischen den HOMO-Niveaus von NPD und 1a, wodurch der Lochtransport in die Emissionsschicht erleichtert werden soll. In einem kombinatorischen Experiment werden verschiedene Schichtdicken (20 nm, 26 nm) für die Emissionsschicht aufgedampft, die aus 15 % Flrpic in 1a als Matrix besteht. Eine 40 nm dicke BAlq=Schicht bildet die lochblockierende Elektronenleiterschicht. Abschließend werden 1 nm Lithiumfluorid und 200 nm Aluminium als Elektrodenmaterial aufgebracht.

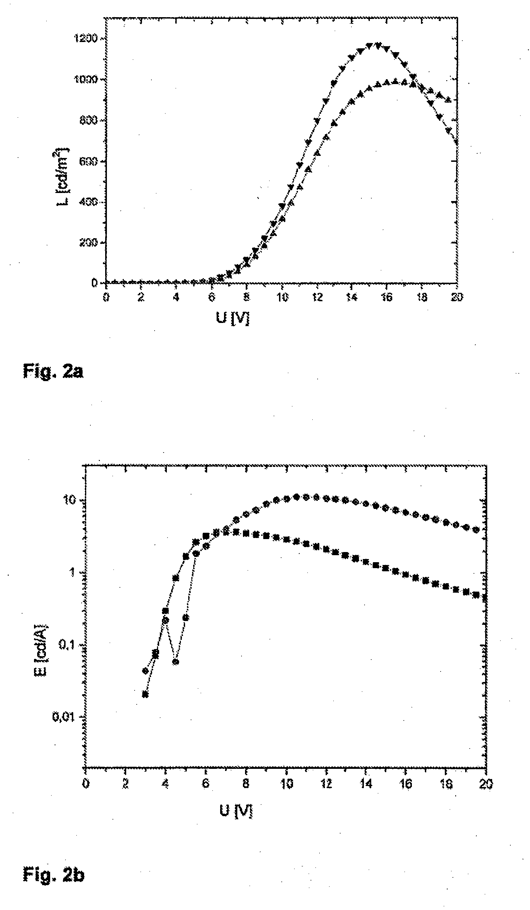

Die Leuchtdichte und die Effizienz der OLED sind in Figur 2a und Figur 2b für Schichtdicken der Emissionsschicht von 20 nm und 26 nm dargestellt.

In Figur 2a bedeuten:
- L [cd/m²]: Leuchtdichte in cd/m²
- U [V]: angelegte Spannung in V

Die Kurve mit den umgekehrten Dreiecken (Spitze nach unten) gibt die Daten für eine 20 nm dicke Emissionsschicht an und die Kurve mit den Dreiecken (Spitze nach oben) gibt die Daten für eine 26 nm dicke Emissionsschicht an.

In Figur 2b bedeuten:
- E [cd/A]: Effizienz in cd/A
- U [V]: angelegte Spannung in V

Die Kurve mit den Quadraten gibt die Daten für eine 20 nm dicke Emissionsschicht an und die Kurve mit den Kreisen gibt die Daten für eine 26 nm dicke Emissionsschicht an.

### II.3 Ergebnis

Die OLED mit 20 nm dicker Emissionsschicht zeigt eine etwas höhere Leuchtdichte von 1200 cd/m² (15 V, 100 mA/cm²) im Vergleich zu 980 cd/m² (15 V, 12 mA/cm²) im Falle der 26 nm dicken Emissionsschicht. Bei 26 nm dicker Emissionsschicht wird ein Effizienzmaximum von 11.0 cd/A bei 10.5 V und einer Leuchtdichte von 500 cd/m² erreicht. Die höchste Effizienz von 3.6 cd/A tritt bei der 20 nm dicken Emissionsschicht bei 7.5 V und einer Leuchtdichte von nur 50 cd/m² auf.

## Patentansprüche

1. Organische Leuchtdiode enthaltend mindestens eine cyclische Phosphazenverbindung der allgemeinen Formel (I) 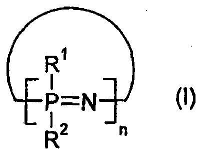 worin bedeuten:
n 3 bis 8, bevorzugt 3 oder 4, besonders bevorzugt 3;
R¹, R² unabhängig voneinander substituiertes oder unsubstituiertes Aryl, substituiertes oder unsubstituiertes Heteroaryl, substituiertes oder unsubstituiertes Alkyl, substituiertes oder unsubstituiertes Aryloxy, substituiertes oder unsubstituiertes Alkyloxy, unsubstituiertes oder substituiertes Cycloalkyl, unsubstituiertes oder substituiertes Heterocycloalkyl, unsubstituiertes oder substituiertes Alkenyl, unsubstituiertes oder substituiertes Alkinyl, unsubstituiertes oder substituiertes Aralkyl oder F, bevorzugt unabhängig voneinander substituiertes oder unsubstituiertes Aryl, substituiertes oder unsubstituiertes Heteroaryl, substituiertes oder unsubstituiertes Alkyl, substituiertes oder unsubstituiertes Aryloxy, substituiertes oder unsubstituiertes Alkyloxy, wobei R¹ und R² in den n Gruppen jeweils gleich oder verschieden sein können;
oder
die vorstehend genannten Reste R¹ und R² sind unabhängig voneinander in den n Gruppen der allgemeinen Formel (I) über eine Bindung, eine C₁-C₆-Alkylenbrücke, eine Arylenbrücke oder eine C₂-C₆-Alkenylenbrücke miteinander verknüpft, so dass die Reste R¹ und R² gemeinsam mit dem P-Atom, mit dem sie verknüpft sind, einen Cyclus bilden;
dadurch gekennzeichet, dass mindestens einer der in den n Gruppen vorliegenden Reste R¹ oder R² mit mindestens einem der P-Atome der cyclischen Phosphazene der Formel (I) nicht über ein Sauerstoffatom verknüpft ist.

2. Organische Leuchtdiode nach Anspruch 1, **dadurch gekennzeichnet, dass** der mindestens eine in den n Gruppen vorliegende Rest R¹ oder R² mit mindestens einem der P-Atome der cyclischen Phosphazenverbindungen der Formel (I) über ein Kohlenstoffatom verknüpft ist.

3. Organische Leuchtdiode nach Anspruch 1 oder 2, **dadurch gekennzeichnet, dass**
n **3 bedeutet.**

4. Organische Leuchtdiode nach einem der Ansprüche 1 bis 3, **dadurch gekennzeichnet, dass** der mindestens eine in den n Gruppen vorliegende Rest R¹ oder R², der mit mindestens einem der P-Atome der cyclischen Phosphazenverbindungen der Formel (I) nicht über ein Sauerstoffatom verknüpft ist, ein unsubstituierter oder substituierter Arylrest oder ein unsubstituierter oder substituierter Heteroarylrest ist.

5. Organische Leuchtdiode nach einem der Ansprüche 1 bis 4, **dadurch gekennzeichnet, dass**
R¹, R² unabhängig voneinander substituiertes oder unsubstituiertes Aryl oder substituiertes oder unsubstituiertes Heteroaryl bedeuten, wobei R¹ und R² in den n Gruppen jeweils gleich oder verschieden sein können.

6. Organische Leuchtdiode nach einem der Ansprüche 2 bis 5, **dadurch gekennzeichnet, dass** die cyclischen Phosphazenverbindungen die Formel (Ia) aufweisen 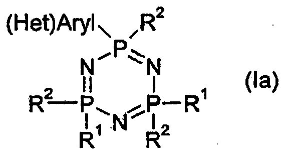 worin bedeuten
(Het)Aryl unsubstituiertes oder substituiertes C₆₋₁₄-Aryl, z. B. Phenyl, C₁-C₆-Alkylphenyl, C₁-C₆-Dialkylphenyl, C₁-C₆-Alkoxyphenyl oder C₁-C₆-Dialkoxyphenyl, oder unsubstituiertes oder substituiertes Heteroaryl, das 5 bis 14 Ringatome aufweist und über ein Kohlenstoffatom mit dem P des Phosphazenrings verknüpft ist, z. B. unsubstituiertes oder substituiertes Carbazolyl, unsubstituiertes oder substituiertes Azacarbazolyl oder unsubstituiertes oder substituiertes Pyridyl;
R¹, R² unabhängig voneinander substituiertes oder unsubstituiertes Aryl, substituiertes oder unsubstituiertes Heteroaryl, substituiertes oder unsubstituiertes Alkyl, substituiertes oder unsubstituiertes Aryloxy, substituiertes oder unsubstituiertes Alkyloxy, wobei R¹ und R² jeweils gleich oder verschieden sein können, bevorzugt unabhängig voneinander substituiertes oder unsubstituiertes Aryl oder substituiertes oder unsubstituiertes Heteroaryl, wobei R¹ und R² jeweils gleich oder verschieden sein können; besonders bevorzugt unsubstituiertes oder substituiertes C₆₋₁₄-Aryl, z. B. Phenyl, C₁-C₆-Alkylphenyl, C₁-C₆-Dialkylphenyl, C₁-C₆-Alkoxyphenyl oder C₁-C₆-Dialkoxyphenyl oder unsubstituiertes oder substituiertes Heteroaryl das 5 bis 14 Ringatome aufweist, z. B. oder unsubstituiertes oder substituiertes Pyridyl, unsubstituiertes oder substituiertes Carbazolyl, unsubstituiertes oder substituiertes Azacarbazolyl wobei R¹ und R² jeweils gleich oder verschieden sein können;
oder
die vorstehend genannten Reste R¹ und R² sind unabhängig voneinander über eine Bindung, eine C₁-C6-Alkylenbrücke, bevorzugt eine C₁-C₂-Alkylenbrücke, eine Arylenbrücke, bevorzugt eine 1,2-Phenylenbrücke, oder eine C₂-C₆-Alkenylenbrücke, bevorzugt eine eine C₂-C₄-Alkenylenbrücke, miteinander verknüpft, so dass die Reste R¹ und R² gemeinsam mit dem P-Atom, mit dem sie verknüpft sind, einen Cyclus bilden; bevorzugt bilden R¹ und R² - in dem Fall, wenn sie miteinander verknüpft sind - gemeinsam unabhängig voneinander in den n Gruppen der allgemeinen Formel (I) eine der folgenden Gruppen: 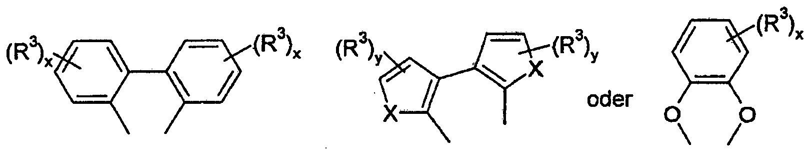
worin bedeuten:
R³ unabhängig voneinander substituiertes oder unsubstituiertes Aryl, substituiertes oder unsubstituiertes Heteroaryl, substituiertes oder unsubstituiertes Alkyl, substituiertes oder unsubstituiertes Aryloxy, substituiertes oder unsubstituiertes Alkyloxy, unsubstituiertes oder substituiertes Cycloalkyl, unsubstituiertes oder substituiertes Heterocycloalkyl, unsubstituiertes oder substituiertes Alkenyl, unsubstituiertes oder substituiertes Alkinyl, unsubstituiertes oder substituiertes Aralkyl oder Halogen, bevorzugt F oder Pseudohalogen, bevorzugt CN, bevorzugt unabhängig voneinander substituiertes oder unsubstituiertes Aryl, substituiertes oder unsubstituiertes Heteroaryl, substituiertes oder unsubstituiertes Alkyl, substituiertes oder unsubstituiertes Aryloxy, substituiertes oder unsubstituiertes Alkyloxy oder F oder CN;
x unabhängig voneinander 0, 1, 2, 3 oder 4, bevorzugt 0, 1 oder 2;
y unabhängig voneinander 0, 1 oder 2, bevorzugt 0 oder 1.

7. Organische Leuchtdiode nach Anspruch 6, **dadurch gekennzeichnet, dass** alle Reste R¹, R² und (Het)Aryl in den cyclischen Phosphazenen der Formel (la) dieselbe Bedeutung aufweisen.

8. Organische Leuchtdiode nach einem der Ansprüche 1 bis 7, **dadurch gekennzeichnet, dass** die cyclischen Phosphazene als Matrixmaterial in der Lichtemittierenden Schicht und/oder als Loch-/Excitonenblockermaterial und/oder Elektronenleiter, besonders bevorzugt als Matrixmaterial in der Licht-emittierenden Schicht und/oder Loch-/Excitonenblocker eingesetzt werden.

9. Organische Leuchtdiode nach einem der Ansprüche 1 bis 8, **dadurch gekennzeichnet, dass** das Emissionsmaximum der organischen Leuchtdiode zwischen 400 und 500 nm liegt.

10. Organische Leuchtdiode nach einem der Ansprüche 1 bis 9, enthaltend eine Licht-emittierende Schicht aufgebaut aus mindestens einem Matrixmaterial und mindestens einem Emittermaterial, **dadurch gekennzeichnet, dass** das mindestens eine Matrixmaterial mindestens eine cyclische Phosphazenverbindung gemäß einem der Ansprüche 1 bis 7 enthält.

11. Verwendung von cyclischen Phosphazenverbindungen gemäß einem der Ansprüche 1 bis 7 in organischen Leuchtdioden.

12. Vorrichtung ausgewählt aus der Gruppe bestehend aus stationären Bildschirmen wie Bildschirmen von Computern, Fernsehern, Bildschirmen in Druckern, Küchengeräten sowie Reklametafeln, Beleuchtungen, Hinweistafeln und mobilen Bildschirmen wie Bildschirmen in Handys, Laptops, Digitalkameras, Fahrzeugen sowie Zielanzeigen an Bussen und Bahnen enthaltend mindestens eine organische Leuchtdiode gemäß einem der Ansprüche 1 bis 10.

## Claims

1. An organic light-emitting diode comprising at least one cyclic phosphazene compound of the general formula (I) 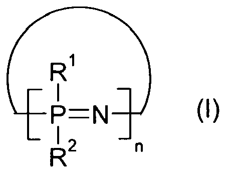 in which:
n is from 3 to 8, preferably 3 or 4, more preferably 3;
R¹, R² are each independently substituted or unsubstituted aryl, substituted or unsubstituted heteroaryl, substituted or unsubstituted alkyl, substituted or unsubstituted aryloxy, substituted or unsubstituted alkyloxy, unsubstituted or substituted cycloalkyl, unsubstituted or substituted heterocycloalkyl, unsubstituted or substituted alkenyl, unsubstituted or substituted alkynyl, unsubstituted or substituted aralkyl or F, preferably each independently substituted or unsubstituted aryl, substituted or unsubstituted heteroaryl, substituted or unsubstituted alkyl, substituted or unsubstituted aryloxy, substituted or unsubstituted alkyloxy, where R¹ and R² in the n groups may each be the same or different;
or
the aforementioned R¹ and R² radicals are each independently, in the n groups of the general formula (I), joined to one another via a bond, a C₁-C₆-alkylene bridge, an arylene bridge or a C₂-C₆-alkenylene bridge, such that the R¹ and R² radicals together with the phosphorus atom to which they are joined form a cycle;
wherein at least one of the R¹ and R² radicals present in the n groups is joined to at least one of the phosphorus atoms of the cyclic phosphazenes of the formula (I) not via an oxygen atom.

2. The organic light-emitting diode according to claim 1, wherein the at least one R¹ or R² radical present in the n groups is joined to at least one of the phosphorus atoms of the cyclic phosphazene compounds of the formula (I) via a carbon atom.

3. The organic light-emitting diode according to claim 1 or 2, wherein
n is 3.

4. The organic light-emitting diode according to any one of claims 1 to 3, wherein the at least one R¹ or R² radical which is present in the n groups and is joined to at least one of the phosphorus atoms of the cyclic phosphazene compounds of the formula (I) not via an oxygen atom is an unsubstituted or substituted aryl radical or an unsubstituted or substituted heteroaryl radical.

5. The organic light-emitting diode according to any one of claims 1 to 4, wherein
R¹, R² are each independently substituted or unsubstituted aryl or substituted or unsubstituted heteroaryl, where R¹ and R² in the n groups may each be the same or different.

6. The organic light-emitting diode according to any one of claims 2 to 5, wherein the cyclic phosphazene compounds have the formula (Ia) 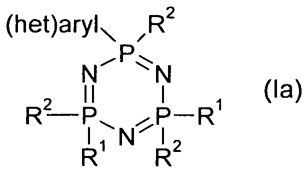 in which
(het) aryl is unsubstituted or substituted C₆₋₁₄-aryl, e.g. phenyl, C₁-C₆-alkylphenyl, C₁-C₆-dialkylphenyl, C₁-C₆-alkoxyphenyl or C₁-C₆-dialkoxyphenyl, or unsubstituted or substituted heteroaryl which has from 5 to 14 ring atoms and is joined to the P of the phosphazene ring via a carbon atom, e.g. unsubstituted or substituted carbazolyl, unsubstituted or substituted azacarbazolyl or unsubstituted or substituted pyridyl;
R¹, R² are each independently substituted or unsubstituted aryl, substituted or unsubstituted heteroaryl, substituted or unsubstituted alkyl, substituted or unsubstituted aryloxy, substituted or unsubstituted alkyloxy, where R¹ and R² may each be the same or different and are preferably each independently substituted or unsubstituted aryl or substituted or unsubstituted heteroaryl, where R¹ and R² may each be the same or different and are more preferably unsubstituted or substituted C₆₋₁₄-aryl, e.g. phenyl, C₁-C₆-alkylphenyl, C₁-C₆-dialkylphenyl, C₁-C₆-alkoxyphenyl or C₁-C₆-dialkoxyphenyl or unsubstituted or substituted heteroaryl which has from 5 to 14 ring atoms, e. g. unsubstituted or substituted pyridyl, unsubstituted or substituted carbazolyl, unsubstituted or substituted azacarbazolyl, where R¹ and R² may each be the same or different;
or
the aforementioned R¹ and R² radicals are each independently joined to one another via a bond, a C₁-C₆-alkylene bridge, preferably a C₁-C₂-alkylene bridge, an arylene bridge, preferably a 1,2-phenylene bridge, or a C₂-C₆-alkenylene bridge, preferably a C₂-C₄-alkenylene bridge, such that the R¹ and R² radicals together with the phosphorus atom to which they are joined form a cycle; R¹ and R² preferably form - in the case where they are joined to one another - together independently, in the n groups of the general formula (I), one of the following groups: 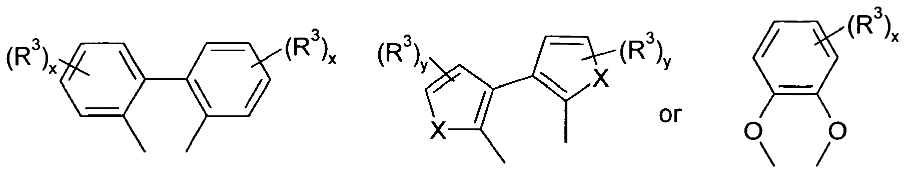
in which:
R³ are each independently substituted or unsubstituted aryl, substituted or unsubstituted heteroaryl, substituted or unsubstituted alkyl, substituted or unsubstituted aryloxy, substituted or unsubstituted alkyloxy, unsubstituted or substituted cycloalkyl, unsubstituted or substituted heterocycloalkyl, unsubstituted or substituted alkenyl, unsubstituted or substituted alkynyl, unsubstituted or substituted aralkyl or halogen, preferably F, or pseudohalogen, preferably CN, preferably each independently substituted or unsubstituted aryl, substituted or unsubstituted heteroaryl, substituted or unsubstituted alkyl, substituted or unsubstituted aryloxy, substituted or unsubstituted alkyloxy or F or CN;
x are each independently 0, 1, 2, 3 or 4, preferably 0, 1 or 2;
y are each independently 0, 1 or 2, preferably 0 or 1.

7. The organic light-emitting diode according to claim 6, wherein all R¹, R² and (het)aryl radicals in the cyclic phosphazenes of the formula (Ia) are defined identically.

8. The organic light-emitting diode according to any one of claims 1 to 7, wherein the cyclic phosphazenes are used as matrix material in the light-emitting layer and/or as hole/exciton blocker material and/or electron conductors, more preferably as matrix material in the light-emitting layer and/or hole/exciton blockers.

9. The organic light-emitting diode according to any one of claims 1 to 8, wherein the emission maximum of the organic light-emitting diode is between 400 and 500 nm.

10. The organic light-emitting diode according to any one of claims 1 to 9, comprising a light-emitting layer formed from at least one matrix material and at least one emitter material, wherein the at least one matrix material comprises at least one cyclic phosphazene compound according to any one of claims 1 to 7.

11. The use of cyclic phosphazene compounds according to any one of claims 1 to 7 in organic light-emitting diodes.

12. A device selected from the group consisting of stationary visual display units such as visual display units of computers, televisions, visual display units in printers, kitchen appliances and advertising panels, illuminations, information panels and mobile visual display units such as visual display units in cellphones, laptops, digital cameras, vehicles and destination displays on buses and trains, comprising at least one organic light-emitting diode according to any one of claims 1 to 10.

## Revendications

1. Diode électroluminescente organique, contenant au moins un composé cyclique de phosphazène de formule générale (I) 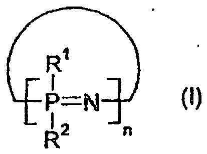 où :
n vaut 3 à 8, de préférence 3 ou 4, de manière particulièrement préférée 3 ;
R¹, R² représentent, indépendamment l'un de l'autre, aryle substitué ou non substitué, hétéroaryle substitué ou non substitué, alkyle substitué ou non substitué, aryloxy substitué ou non substitué, alkyloxy substitué ou non substitué, cycloalkyle substitué ou non substitué, hétérocycloalkyle substitué ou non substitué, alcényle substitué ou non substitué, alcynyle substitué ou non substitué, aralkyle substitué ou non substitué ou F, de préférence, indépendamment l'un de l'autre, aryle substitué ou non substitué, hétéroaryle substitué ou non substitué, alkyle substitué ou non substitué, aryloxy substitué ou non substitué, alkyloxy substitué ou non substitué, R¹ et R² pouvant à chaque fois être identiques ou différents dans les n groupes ; ou
les radicaux susmentionnés R¹ et R², indépendamment l'un de l'autre, dans les n groupes de formule générale (I), sont reliés l'un à l'autre via une liaison, un pont C₁-C₆-alkylène, un pont arylène ou un pont C₂-C₆-alcénylène, de telle sorte que les radicaux R¹ et R² forment un cycle, ensemble avec l'atome de P auquel ils sont reliés ;
**caractérisée en ce qu'** au moins un des radicaux R¹ ou R² se trouvant dans les n groupes n'est pas relié via un atome d'oxygène à au moins un des atomes de P du phosphazène cyclique de formule (I).

2. Diode électroluminescente organique selon la revendication 1, **caractérisée en ce que** ledit au moins un radical R¹ ou R² se trouvant dans les n groupes est relié à au moins un des atomes de P des composés cycliques de phosphazène de formule (I) via un atome de carbone.

3. Diode électroluminescente organique selon la revendication 1 ou 2, **caractérisée en ce que**
n vaut 3.

4. Diode électroluminescente organique selon l'une quelconque des revendications 1 à 3, **caractérisée en ce que** ledit au moins un radical R¹ ou R² se trouvant dans les n groupes, qui n'est pas relié à au moins un des atomes de P des composés cycliques de phosphazène de formule (I) via un atome d'oxygène, est un radical aryle substitué ou non substitué ou un radical hétéroaryle substitué ou non substitué.

5. Diode électroluminescente organique selon l'une quelconque des revendications 1 à 4, **caractérisée en ce que**
R¹, R² signifient, indépendamment l'un de l'autre, aryle substitué ou non substitué, ou hétéroaryle substitué ou non substitué, R¹ et R² dans les n groupes pouvant à chaque fois être identiques ou différents.

6. Diode électroluminescente organique selon l'une quelconque des revendications 2 à 5, **caractérisée en ce que** les composés cycliques de phosphazène présentent la formule (Ia) 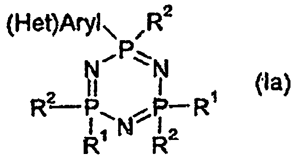 où
(Het)Aryl signifie C₆₋₁₄-aryle substitué ou non substitué, par exemple phényle, C₁-C₆-alkylphényle, C₁-C₆-dialkylphényle, C₁-C₆-alcoxyphényle ou C₁-C₆-dialcoxyphényle ; ou hétéroaryle substitué ou non substitué, qui présente 5 à 14 atomes de cycle et qui est relié via un atome de carbone à l'atome P du cycle phosphazène, par exemple carbazolyle substitué ou non substitué, azacarbazolyle substitué ou non substitué ou pyridyle substitué ou non substitué ;
R¹, R² signifient, indépendamment l'un de l'autre, aryle substitué ou non substitué, hétéroaryle substitué ou non substitué, alkyle substitué ou non substitué, aryloxy substitué ou non substitué, alkyloxy substitué ou non substitué, R¹ et R² pouvant à chaque fois être identiques ou différents, de préférence, il signifient, indépendamment l'un de l'autre, aryle substitué ou non substitué ou hétéroaryle substitué ou non substitué, R¹ et R² pouvant à chaque fois être identiques ou différents ; de manière particulièrement préférée, il signifient C₆₋₁₄-aryle substitué ou non substitué, par exemple phényle, C₁-C₆-alkylphényle, C₁-C₆-dialkylphényle, C₁-C₆-alcoxyphényle ou C₁-C₆-dialcoxyphényle ou hétéroaryle substitué ou non substitué, qui présente 5 à 14 atomes de cycle, par exemple pyridyle substitué ou non substitué, carbazolyle substitué ou non substitué, azacarbazolyle substitué ou non substitué, R¹ et R² pouvant à chaque fois être identiques ou différents ; ou
les radicaux R¹ et R² susmentionnés sont reliés l'un à l'autre, indépendamment l'un de l'autre, via une liaison, un pont C₁-C₆-alkylène, de préférence un pont C₁-C₂-alkylène, un pont arylène, de préférence un pont 1,2-phénylène, ou un pont C₂-C₆-alcénylène, de préférence un pont C₂-C₄-alcénylène, de manière telle que les radicaux R¹ et R² forment un cycle, ensemble avec l'atome de P auquel ils sont liés ; de préférence R¹ et R² forment ensemble - dans le cas où ils sont liés l'un à l'autre - indépendamment l'un de l'autre, dans les n groupes de formule générale (I), un des groupes suivants : 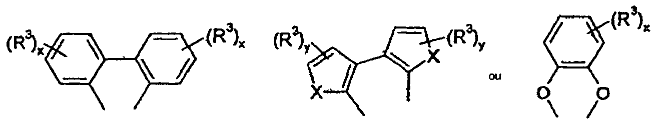
où :
R³ représente, indépendamment l'un de l'autre, aryle substitué ou non substitué, hétéroaryle substitué ou non substitué, alkyle substitué ou non substitué, aryloxy substitué ou non substitué, alkyloxy substitué ou non substitué, cycloalkyle substitué ou non substitué, hétérocycloalkyle substitué ou non substitué, alcényle substitué ou non substitué, alcynyle substitué ou non substitué, aralkyle substitué ou non substitué ou halogène, de préférence F ou pseudohalogène, de préférence CN, de préférence, indépendamment l'un de l'autre, aryle substitué ou non substitué, hétéroaryle substitué ou non substitué, alkyle substitué ou non substitué, aryloxy substitué ou non substitué, alkyloxy substitué ou non substitué ou F ou CN ;
x vaut, indépendamment l'un de l'autre, 0, 1, 2, 3 ou 4, de préférence 0, 1 ou 2 ;
y vaut, indépendamment l'un de l'autre, 0, 1 ou 2, de préférence 0 ou 1.

7. Diode électroluminescente organique selon la revendication 6, **caractérisée en ce que** tous les radicaux R¹, R² et (Het)Aryl dans les phosphazènes cycliques de formule (Ia) présentent la même signification.

8. Diode électroluminescente organique selon l'une quelconque des revendications 1 à 7, **caractérisée en ce que** les phosphazènes cycliques sont utilisés comme matériau de matrice dans la couche émettrice de lumière et/ou comme matériau de blocage des trous/excitons et/ou comme conducteur électronique, de manière particulièrement préférée comme matériau de matrice dans la couche émettrice de lumière et/ou matériau de blocage des trous/excitons.

9. Diode électroluminescente organique selon l'une quelconque des revendications 1 à 8, **caractérisée en ce que** le maximum d'émission de la diode électroluminescente organique se situe entre 400 et 500 nm.

10. Diode électroluminescente organique selon l'une quelconque des revendications 1 à 9, contenant une couche émettrice de lumière, formée d'au moins un matériau de matrice et d'au moins un matériau émetteur, **caractérisée en ce que** ledit au moins un matériau de matrice contient au moins un composé cyclique du phosphazène selon l'une quelconque des revendications 1 à 7.

11. Utilisation de composés cycliques de phosphazène selon l'une quelconque des revendications 1 à 7 dans des diodes électroluminescentes organiques.

12. Dispositif choisi dans le groupe constitué par les écrans stationnaires, tels que les écrans d'ordinateurs, de téléviseurs, les écrans dans les imprimantes, les appareils de cuisine ainsi que les panneaux publicitaires, les éclairages, les panneaux indicateurs et les écrans mobiles, tels que les écrans dans les téléphones portables, les ordinateurs portables, les caméras digitales, les véhicules ainsi que les affichages de destination sur les bus et les trains contenant au moins une diode électroluminescente organique selon l'une quelconque des revendications 1 à 10.
